# EUROPÄISCHE PATENTSCHRIFT

(11) **EP 2 911 738 B1**
(45) Veröffentlichungstag und Bekanntmachung des Hinweises auf die Patenterteilung: **31.01.2018**
(21) Anmeldenummer: 13811775.9
(22) Anmeldetag: 24.10.2013
(51) Int. Cl.: A61N 1/39, A61B 5/06, A61M 11/00, A61N 1/08, A61N 1/05, A61B 17/34

(54) **VORRICHTUNG UND MULTIFUNKTIONSGERÄT ZUR BEAUFSCHLAGUNG VON KÖRPERGEWEBE MIT STROMIMPULSEN**
DEVICE AND MULTI-FUNCTION DEVICE FOR APPLYING ELECTRICAL PULSES TO BODILY TISSUE
DISPOSITIF ET APPAREIL MULTIFONCTIONS POUR APPLIQUER DES IMPULSIONS DE COURANT À UN TISSU CORPOREL

(30) Priorität: 27.10.2012 DE 102012021136
(43) Veröffentlichungstag der Anmeldung: 02.09.2015
(73) Patentinhaber: L&S Medtec GbR, 89155 Erbach (DE)
(72) Erfinder: LIU, Yuefei, 89231 Neu-Ulm (DE); SIMON, Hans-Jörg, 89155 Erbach (DE)
(74) Vertreter: Spengler, Robert
(86) Internationale Anmeldenummer: PCT/DE2013/000633
(87) Internationale Veröffentlichungsnummer: WO 2014/063679

(56) Entgegenhaltungen:
- US-A- 5 845 646
- US-A1- 2002 120 260
- US-A1- 2012 130 169
- US-B1- 6 176 856

## Beschreibung

Die vorliegende Erfindung betrifft eine Vorrichtung zur Beaufschlagung von vorgegebenem Körpergewebe mit Stromimpulsen, die an Körperoberflächen von Organismen anlegbar ist, sowie ein Multifunktionsgerät mit einer solchen Vorrichtung. Bei dem vorgegebenen Körpergewebe kann es sich um inneres Muskelgewebe oder innere Organe lebendiger Organismen, und zwar sowohl von Menschen als auch von Tieren oder Pflanzen, aber auch um inneres Muskelgewebe oder innere Organe von toten Organismen, wie menschlichen Leichen oder Tierkadavern, handeln.

An Körpergewebe toter Organismen werden Stromimpulse für gewöhnlich zu Forschungszwecken abgegeben, während die Abgabe von Stromimpulsen an Körpergewebe lebendiger Organismen zumeist aus medizinischen Gründen erforderlich ist. Ein bekanntes Beispiel für eine Vorrichtung zur Beaufschlagung von inneren Organen mit Stromimpulsen aus medizinischen Gründen sind Herzschrittmacher, welche die Muskeln des Herzens von Patienten mittels elektrischer Impulse stimulieren und dadurch zur Kontraktion anregen. Ferner sind zur Behandlung von Herzrhythmusstörungen wie Kammerflimmern Defibrillatoren mit großflächigen Elektroden bekannt, welche auf dem Brustkorb von Patienten angeordnet werden, um durch die Brust Stromstöße an das Herz abzugeben. Defibrillatoren spielen in der Notfallmedizin eine bedeutende Rolle. Zahlreiche Defibrillatoren werden dementsprechend nicht nur in Notfallaufnahmen von Krankenhäusern und in Fahrzeugen des Rettungsdienstes sondern auch an vielen öffentlich zugänglichen Orten bereitgehalten, wo sie im Notfall für eine Anwendung auch durch medizinische Laien zur Verfügung stehen.

Insbesondere in Fällen eines als Asystolie bezeichneten Stillstandes der elektrischen und mechanischen Herzaktion, der bei Ausbleiben einer rechtzeitigen Behandlung innerhalb weniger Minuten zum Tode führt, bleiben Defibrillatoren jedoch wirkungslos. Im Gegensatz zum Kammerflimmern, bei der die Defibrillation mittels eines Defibrillators eine spezifische und effektive Hilfsmaßnahme darstellt, sind Asystolien für einen weit größeren Anteil an Herztoden ursächlich. Gleichwohl mangelt es hier an einer effektiven durchführbaren spezifischen Behandlung. Heutzutage ist man daher notgedrungen auf die Anwendung extrakorporaler Herzdruckmassagen als einziger Möglichkeit zur Notfallbehandlung von Asystolien angewiesen. Diese erfordern jedoch ein gewisses Maß an Geschicklichkeit und können von ungeübten Laien nur mit Schwierigkeiten durchgeführt werden. Aber selbst von geübtem Fachpersonal durchgeführte Herzdruckmassagen haben eine sehr niedrige Erfolgsrate von unter 5% und bringen zudem in der Regel unerwünschte Nebenwirkungen für den Patienten mit sich bis hin zu erheblichen Beeinträchtigungen seiner Gesundheit wie inneren Organverletzungen oder Rippenfrakturen. Aus diesen Gründen ist insbesondere für die Notfallbehandlung von Asystolien eine Möglichkeit der intrakardialen Schrittmacheraktion wünschenswert, was jedoch nicht ohne ein vorheriges Einbringen einer Elektrode in das Herz möglich ist. Nun sind derzeit verschiedene Verfahren bekannt, mit denen sich diverse medizinische Gerätschaften in inneres Körpergewebe oder innere Organe einbringen oder in der Nähe von inneren Organen lebender oder toter Organismen positionieren lassen. Beispielsweise ist aus der US 6,309,370 B1 ein Katheter aus einem biegsamen Material bekannt, der dazu vorgesehen ist, automatisch oder manuell über eine Arterie in eine Herzkammer eingeführt zu werden. Hierzu ist der Katheter mit einem Positionssensor zur Bestimmung der Position eines vorderen Endabschnitts des Katheters relativ zum Herzen und zur Herzkammer versehen. Ein hierfür geeigneter Positionssensor wiederum ist in der WO 96/05768 A1 offenbart.
Die US 6,176,856 B1 offenbart ein Kathetersystem mit einem äußeren Katheter und einem inneren Katheter, der innerhalb des äußeren Katheters beweglich aufgenommen ist. Eine Eindringeinrichtung ist an einem vorderen Ende des inneren Katheters angeordnet. Im Inneren der Eindringeinrichtung 22 ist ein Energieversorgungsmittel 28 vorgesehen. Das Gerät dient zum kontrollierten Einbringen einer Nadel in das Epikard um die sogenannte transmyokardiale Revaskularisation durchzuführen.

Dokument US 2002/0120260 A1 offenbart eine Vorrichtung gemäß erstem Teil des unabhängigen Anspruchs 1.

Alle bekannten Verfahren zum Einbringen von medizinischem Gerät in Organismen sind jedoch mit einem großen Zeitaufwand verbunden und sie können fast nur von medizinischem Fachpersonal mit entsprechender Ausrüstung ausgeführt werden. Auch das Einbringen einer Elektrode in das Herz, um mit ihr intrakardiale Schrittmacheraktionen durchzuführen, ist derzeit nur mit einnem komplizierten und langwierigen operativen Eingriff möglich. Aus diesen Gründen gibt es derzeit auch keine Möglichkeit zur Notfallbehandlung von Asystolien, bei denen die Zeit ein kritischer Faktor ist, mittels intrakardialer Schrittmacheraktionen, und zwar weder für geübte Ärzte oder Nothelfer und erst recht nicht für medizinische Laien.

Angesichts dieser Problematik ist es die Aufgabe der vorliegenden Erfindung, eine Vorrichtung und ein Multifunktionsgerät mit einer solchen Vorrichtung zu schaffen, die es selbst medizinisch ungeübten Laien ermöglichen, Körpergewebe von Organismen rasch mit Stromimpulsen zu versorgen.

Diese Aufgabe wird durch die Vorrichtung mit den Merkmalen des Anspruchs 1 und durch das Multifunktionsgerät mit den Merkmalen des Anspruchs 13 gelöst. Bevorzugte Ausführungen sind Gegenstand der abhängigen Ansprüche.

Um vorgegebenes Körpergewebe eines Organismus, beispielsweise ein inneres Organ wie das Herz, mit Stromimpulsen zu beaufschlagen oder zu versorgen, wird die erfindungsgemäße Vorrichtung von einem Benutzer zunächst an einer Stelle an die Körperoberfläche des Organismus angelegt, die vorzugsweise einer endgültigen Zielposition für die Vorrichtung möglichst nahe ist. Die Positionserfassungseinrichtung erfasst sodann die Position wenigstens eines Teils der an der Körperoberfläche anliegenden Vorrichtung in Bezug auf das mit Stromimpulsen zu versorgende Körpergewebe oder einen Teil desselben. Bei besagtem Teil der Vorrichtung handelt es sich bevorzugt um die Eindringeinrichtung oder einen Teil davon, wie zum Beispiel einen vorderen Endabschnitt der Eindringeinrichtung oder die Elektrode. Sofern es sich bei dem mit Stromimpulsen zu beaufschlagenden Körpergewebe um das Herz des Organismus handelt, so kann die Positionserfassungseinrichtung die Position der Vorrichtung oder eines Teils derselben in Bezug auf beispielsweise eine der Herzkammern erfassen. Die erfasste Position wird von der Positionserfassungseinrichtung überprüft und in Abhängigkeit von dieser Überprüfung wird vom Signalausgabemittel wenigstens ein Signal ausgegeben. Wird die erfasste Position von der Positionserfassungseinrichtung zum Überführen der Eindringeinrichtung in die Endposition beim Überprüfen als geeignet eingestuft, so gibt das Signalausgabemittel beispielsweise wenigstens ein erstes Signal aus. Das Signalausgabemittel kann ferner dazu eingerichtet sein, außer dem ersten Signal das ausgegeben wird, wenn die erfasste Position als geeignet eingestuft wird, wenigstens ein zweites Signal auszugeben, wenn die Position als ungeeignet eingestuft wird, wobei die beiden Signale voneinander verschieden sind. Wird die erfasste Position als ungeeignet befunden, so wird je nach Ausführungsform der erfindungsgemäßen Vorrichtung zum Beispiel kein Signal oder es wird wenigstens ein zweites Signal von dem Signalausgabemittel ausgegeben, was dem Benutzer signalisiert, dass es der Eindringeinrichtung in ihrer derzeitigen Position nicht möglich ist, bestimmungsgemäß in den Organismus einzudringen und das Körpergewebe zu kontaktieren, und dass ein Versetzen der Vorrichtung an eine andere Stelle auf der Körperoberfläche notwendig ist. Alternativ kann die Ausgabe wenigstens eines entsprechenden Signals nur für den Fall vorgesehen sein, dass die erfasste Position als ungeeignet angesehen wird, während kein Signal ausgegeben wird, sofern die erfasste Position als geeignet eingestuft wird. Nach Versetzen der Vorrichtung wird nun von der Positionserfassungseinrichtung die jeweils neue Position der Vorrichtung oder des Teils der Vorrichtung in Bezug auf das Körpergewebe erfasst und je nachdem, ob diese neue Position als geeignet eingestuft wird oder nicht, wird ein vorgegebenes Signal vom Signalausgabemittel entweder ausgegeben oder nicht ausgegeben. Wird vom Signalausgabemittel abermals signalisiert, dass die erfasste Position ungeeignet ist, muss der Benutzer die Vorrichtung auf der Suche nach einer Position, an welcher es der Eindringeinrichtung möglich ist, die Endposition zu erreichen, wieder versetzen. Dieser Vorgang wird so lange wiederholt, bis eine geeignete Position gefunden ist und das Signalausgabemittel dem Benutzer auf die jeweils vorgegebene Weise signalisiert, dass in der derzeitigen Position der Vorrichtung die Eindringeinrichtung prinzipiell so in den Organismus eingebracht werden kann, dass der Elektrode ein Kontaktieren des Körpergewebes ermöglicht wird. Nach Überführen der Eindringeinrichtung in die Endposition und Herstellen eines Kontaktes zwischen der Elektrode und dem betreffenden Körpergewebe kann das Körpergewebe nunmehr mit Stromimpulsen beaufschlagt oder versorgt werden, welche die Elektrode an das Körpergewebe abgibt. Auf diese Weise kann die Vorrichtung der vorliegenden Erfindung besonders einfach und selbst von medizinisch ungeübten Laien bedient werden. Die vorliegende Erfindung ermöglicht es Laien insbesondere, die Vorrichtung problemlos auf dem Körper von Patienten korrekt zu platzieren sowie die Eindringeinrichtung in deren Körper zu injizieren und in einen Zustand zu bringen, in welchem die Elektrode mit dem Körpergewebe in Kontakt ist, um nach Herstellen des Kontakts Stromimpulse an das Körpergewebe abzugeben.

Grundsätzlich ist die Art der vom Signalausgabemittel ausgegebenen Signale unerheblich. So kann es sich bei dem Signalausgabemittel um eine oder mehrere einfache Leuchtdioden, einen Bildschirm oder Display oder einen berührungssensitiven Bildschirm oder Berührungsbildschirm bzw. Touchscreen oder einen Lautsprecher handeln. Entsprechend kann es sich bei einem Signal oder bei mehreren Signalen beispielsweise um ein optisches Signal oder ein Lichtsignal oder ein Blinksignal oder eine Textanzeige oder ein akustisches Signal oder eine Sprachausgabe oder ein Vibrationssignal oder eine Kombination zweier oder mehrerer dieser oder anderer Signale handeln.

Es kann ferner eine akustische und/oder eine visuelle Bedienerführung für die erfindungsgemäße Vorrichtung vorgesehen sein. Desweiteren sind sprachgeführte oder menügeführte Ausführungen der Vorrichtung möglich.

Vorteilhafterweise weist die erfindungsgemäße Vorrichtung eine im Wesentlichen flache Deckplatte an einem während des Einsatzes der Vorrichtung von der Körperoberfläche abgewandten Ende auf. Eine flache Deckplatte erlaubt es die Vorrichtung in Fällen, in denen die Behandlung einer Asystolie mittels der Vorrichtung erfolglos bleibt, nichtsdestotrotz für Herzdruckmassagen zu verwenden. Dabei kann die Eindringeinrichtung vor Beginn der Herzdruckmassage von dem oder aus dem Herzen bzw. aus dem Organismus zurückgezogen werden, was jedoch nur durch einen Arzt durchgeführt werden sollte. Es ist aber auch möglich, das Herz auch während der Herzdruckmassage mittels der Vorrichtung mit Stromimpulsen zu versorgen, um die Wirksamkeit der Herzdruckmassage zu erhöhen.

Bevorzugt erkennt die Positionserfassungseinrichtung die erfasste Position dann als geeignet, wenn sich die erfasste Position zudem innerhalb wenigstens eines vorgegebenen Positionierungsbereichs befindet. Das Fallen der erfassten Position in den vorgegebenen Positionierungsbereich wird bei einer solchen Ausführung der Erfindung als Kriterium dafür herangezogen, ob die erfasste Position zum Einbringen der Eindringeinrichtung in den Organismus grundsätzlich geeignet ist oder nicht. Dabei zeichnen sich die einzelnen zum Positionierungsbereich gehörigen Positionen dadurch aus, dass, sofern die Vorrichtung oder deren Teil eine dieser Positionen einnimmt, die Eindringeinrichtung damit prinzipiell in die Lage versetzt ist, ihre bestimmungsgemäße Funktion zu erfüllen und so in den Organismus einzudringen, dass sie die Endposition einnehmen kann, in welchem die Elektrode mit dem Körpergewebe in Kontakt tritt. Eine von der Positionserfassungseinrichtung erfasste Position lässt sich leicht mit dem Positionierungsbereich vergleichen um festzustellen, ob diese in den Positionierungsbereich fällt oder nicht. Wird bei diesem Vergleich festgestellt, dass die erfasste Position in den vorgegebenen Positionierungsbereich fällt, so wird sie als geeignet eingestuft und das Signalausgabemittel reagiert entsprechend. Fällt die erfasste Position hingegen nicht in den vorgegebenen Positionierungsbereich, so wird sie als ungeeignet eingestuft, was vom Signalausgabemittel entsprechend angezeigt wird.

In der Regel ist je nachdem, um welches vorgegebene Körpergewebe es sich handelt, bzw. welches der inneren Organe des Organismus mit Strom zu beaufschlagen ist, die Menge der einzelnen Positionen, welche zum Überführen der Eindringeinrichtung in die Endposition grundsätzlich geeignet sind, verschieden. Somit unterscheiden sich für verschiedene innere Organe des Organismus die jeweiligen Positionierungsbereiche voneinander. Vorteilhafterweise sind der Vorrichtung deshalb einzelnen Organen bestimmter Organismen jeweilige zugeordnete Positionierungsbereiche vorgegeben, die zum Beispiel in einer Speichereinheit der Vorrichtung in Datenform abgespeichert sein können. Diese Positionierungsbereiche können vorab anhand von typischen oder durchschnittlichen Abmessungen für den betreffenden Organismus ermittelt werden, die aus der Vermessung einer Vielzahl individueller Organismen resultieren. Die Vorrichtung weist ferner vorteilhaft eine Auswahlmöglichkeit auf, mit der ein Benutzer der Vorrichtung je nach dem Organ, das mit Stromimpulsen versorgt werden soll, den zugehörigen Positionierungsbereich auswählt, welcher dann dem Vergleich mit der von der Positionserfassungseinrichtung erfassten Position zugrunde gelegt werden soll.

Obwohl beim ersten Anlegen der Vorrichtung an die Körperoberfläche deren relative Lage in Bezug auf das vorgegebene Körpergewebe oder Organ grundsätzlich unerheblich ist, ist es zum Beschleunigen des Platzierungsvorganges vorteilhaft, wenn die Vorrichtung zu Beginn wenigstens ungefähr in relativer Nähe zum betreffenden Organ an den Organismus angelegt wird. Um dem Benutzer die Auswahl einer möglichst geeigneten Stelle auf der Körperoberfläche zum Anlegen der Vorrichtung zu erleichtern bzw. um dafür zu sorgen, dass der Benutzer die Vorrichtung möglichst schon beim ersten Anlegen an einer Stelle platziert, bei der sich die erfasste Position innerhalb des Positionierungsbereichs befindet, weist die Vorrichtung bevorzugt eine grafische Anzeige auf, mit der sich ein Bereich geeigneter Anlagestellen für die Vorrichtung auf dem Körper des Organismus anzeigen lässt.

Alternativ oder zusätzlich zum vorgegebenen Positionierungsbereich können auch andere Kriterien für die Entscheidung herangezogen werden, ob die erfasste Position zum Überführen der Eindringeinrichtung in die Endposition geeignet ist oder nicht, wie zum Beispiel das Vorhandensein von Knochengewebe zwischen der zur Injektion der Eindringeinrichtung vorgesehenen Stelle und dem vorgegebenen Körpergewebe. Da das Vorhandensein von Knochengewebe zwischen der vorgesehenen Injektionsstelle und dem vorgegebenen Körpergewebe das Vordringen der Eindringeinrichtung in den Organismus bis zum Körpergewebe behindert bzw. verhindert, wird die Eindringeinrichtung bevorzugt an einer Stelle in den Organismus eingebracht, von der aus sie auf ihrem Weg bis zum Körpergewebe möglichst nicht von Knochengewebe und anderen Hindernissen wie zum Beispiel Gewebewucherungen behindert wird. Nur wenn festgestellt wird, dass im Falle einer erfassten Position keine derartigen Hindernisse bestehen, wird diese erfasste Position als geeignet zum Einbringen der Eindringeinrichtung in den Organismus eingestuft, ansonsten wird sie als ungeeignet eingestuft.

Bei der Eindringeinrichtung kann es sich im einfachsten Fall um eine Kanüle handeln, die aus Metall oder aus Kunststoff gefertigt ist. Es sind Ausführungsformen der erfindungsgemäßen Vorrichtung möglich, bei denen die Eindringeinrichtung bereits während des Überführens in die Endposition mit dem Körpergewebe in Kontakt kommt sowie Ausführungsformen, bei denen sie erst in der Endposition mit dem Körpergewebe in Kontakt kommt. Die Eindringeinrichtung kann hierbei wenigstens teilweise in das Körpergewebe oder Organ eindringen oder eine Außenwand eines Organs durchstoßen, um wenigstens teilweise oder vollständig in eine Kammer des Organs einzudringen. Bei dem Kontakt zwischen der Elektrode und dem Organ handelt es sich im einfachsten Fall um ein punktuelles Berühren des Organs durch die Elektrode. Ist die Elektrode flächig ausgebildet, so kann sie auch flächig an der Außenseite des Organs anliegen. Sofern die Elektrode dazu vorgesehen ist, das Organ lediglich punktuell zu berühren oder flächig an der Außenseite des Organs anzuliegen, ist es vorteilhaft, wenn die erfindungsgemäße Vorrichtung eine mechatronische Einrichtung aufweist, die für eine dynamische Anpassung oder Nachführung der das Organ kontaktierenden Elektrode sorgt, sobald es zu Kontraktionen oder sonstigen Bewegungen des Organs kommt. Bei anderen Ausführungsformen der Erfindung durchstößt die Elektrode die Außenfläche des Organs und dringt teilweise oder vollständig in das Organ ein. Weist das Organ wenigstens eine innere Kammer auf, so kann die Eindringeinrichtung zum Beispiel eine Außenwand des Organs durchstoßen und in die Kammer eindringen, wo sich die Elektrode an eine innere Wandfläche der Kammer anlegt, um den Kontakt mit dem Organ herzustellen. Dabei kann sich ein Teil der Elektrode bis in die Außenwand hinein erstrecken, um zusätzlich zum Kontakt an der inneren Wandfläche auch an derjenigen Stelle, an der die Außenwand des Organs durchstoßen worden ist, im Inneren der Außenwand mit derselben in Kontakt zu sein, wodurch die Stromübertragung an das Organ infolge der größeren Kontaktfläche verbessert ist. Es sind beliebige Kombinationen dieser Ausführungsformen möglich. Beispielsweise wird eine Ausführung der erfindungsgemäßen Vorrichtung zum Beaufschlagen des menschlichen Herzens mit Stromimpulsen besonders bevorzugt, bei der die Eindringeinrichtung eine Herzkammerwand durchstößt und in eine Herzkammer eintritt, woraufhin die in einem zusammengefalteten Zustand in einem Hohlraum der Eindringeinrichtung angeordnete Elektrode aus diesem ausfährt und sich entfaltet, woraufhin sich die Eindringeinrichtung wieder so weit aus der Herzkammer zurückzieht, dass die entfaltete Elektrode mit einer Innenseite der Herzkammer zur Anlage kommt. Der Zustand, in welchem sich die Eindringeinrichtung befindet, wenn die entfaltete Elektrode an der Innenseite der Herzkammer anliegt und dadurch mit dem Herzen in Kontakt ist, stellt bei dieser Ausführung der vorliegenden Erfindung die Endposition der Eindringeinrichtung dar.

Zur Versorgung der erfindungsgemäßen Vorrichtung und insbesondere der Elektrode, über welche die Stromimpulse an das Körpergewebe abgegeben werden, mit Strom kann die Vorrichtung entweder über eine oder mehrere eigene Stromquellen verfügen oder sie kann wenigstens einen Stromanschluss zum Versorgen der Elektrode mit Strom aufweisen oder sie weist sowohl eine oder mehrere eigene Stromquellen als auch wenigstens einen Stromanschluss auf. Über diesen Stromanschluss kann die Vorrichtung mit einer externen Energie- oder Stromquelle verbindbar sein. Beispielsweise kann die Vorrichtung für den Netzbetrieb ausgelegt sein und über den Stromanschluss mit einem Stromnetz verbindbar sein. Ferner kann die Vorrichtung eine Ausnehmung aufweisen, in welcher der Stromanschluss angeordnet ist und in welche eine oder mehrere Batterien oder Akkumulatoren oder andere Stromquellen einsetzbar sind.

Die Vorrichtung der vorliegenden Erfindung umfasst bevorzugt wenigstens einen Sensor und/oder eine Sensoranordnung und/oder wenigstens einen Ultraschallsensor und/oder wenigstens einen Infrarotsensor und/oder wenigstens einen Kraftsensor und/oder wenigstens einen Tiefensensor und/oder wenigstens einen magnetischen Positionssensor und/oder wenigstens einen Lasersensor und/oder wenigstens eine Laserdiode und/oder wenigstens einen Gassensor und/oder wenigstens einen Hautwiderstandssensor und/oder wenigstens einen Sensor zur Impedanzmessung. Grundsätzlich kann sowohl die Positionserfassungseinrichtung als auch die Eindringeinrichtung mit einem Sensor ausgestattet sein. Es können aber auch Sensoren vorgesehen sein, die von beiden dieser Einrichtungen genutzt werden. So kann die Positionserfassungseinrichtung mit einem oder mehreren Ultraschallsendern und - empfängern als Ultraschallsensoren oder mit Infrarotsensoren ausgestattet sein, um die Position wenigstens eines Teils der Vorrichtung in Bezug auf wenigstens einen Teil eines inneren Organs des Organismus zu erfassen. Ebenso kann die Eindringeinrichtung mit einem oder mehreren Ultraschallsendern und -empfängern oder Ultraschallsensoren oder Infrarotsensoren ausgestattet sein, um mit ihrer Hilfe die Eindringeinrichtung auf ihrem Weg in die Endposition zu überwachen. Alternativ können Ultraschallsensoren oder Infrarotsensoren vorgesehen sein, die sowohl von der Positionserfassungseinrichtung zum Erfassen der Position wenigstens eines Teils der Vorrichtung in Bezug auf wenigstens einen Teil eines inneren Organs des Organismus als auch zum Überwachen des Eindringens der Eindringeinrichtung in den Organismus verwendet werden. Mittels Ultraschallsendern und -empfängern bzw. Ultraschallsensoren und Infrarotsensoren lassen sich innere Organe und insbesondere Teile innerer Organe wie zum Beispiel Hohlräume wie auch der Vorgang bzw. das Voranschreiten des Vordringens der Eindringeinrichtung in den Organismus bildlich erfassen. Sofern die erfindungsgemäße Vorrichtung einen Bildschirm aufweist oder mit einem Bildschirm verbindbar ist, können die mit solchen Sensoren gewonnenen Bilder auf dem Bildschirm angezeigt werden und dem Benutzer die Bedienung der Vorrichtung erleichtern sowie zur Steuerung der Eindringeinrichtung während des Eindringens in den Organismus sowie zur Steuerung des Kontaktierens des Körpergewebes oder Organs durch die Elektrode verwendet werden. Dabei werden besonders bevorzugt ein Infrarotsensor oder ein Ultraschallsensor zur Erfassung einer Position wenigstens eines Teils der an der Körperoberfläche anliegenden Vorrichtung in Bezug auf wenigstens einen Teil des Körpergewebes für die Prüfung, ob diese Position zum Überführen der Eindringeinrichtung in die Endposition geeignet ist, vorgesehen, bzw. es wird ein Ultraschallsensor zum Erfassen der Tiefe, bis zu der die Eindringeinrichtung in den Organismus eingedrungen ist, vorgesehen. Kraftsensoren sind bevorzugt als Teil der Eindringeinrichtung vorgesehen, um mit ihnen das Eindringen der Eindringeinrichtung oder der Elektrode in den Organismus bzw. in das Körpergewebe oder Organ zu steuern. Beispielsweise muss zum Durchdringen der Haut und des äußeren Muskelgewebes von Organismen in der Regel eine merklich höhere Kraft aufgewendet werden als zum Eindringen in ein Organ. Anhand des Verlaufs der beim Eindringen der Eindringeinrichtung in den Organismus aufzuwendenden Kraft bzw. anhand eines Abfallens dieser Kraft lässt sich daher erkennen, dass die Eindringeinrichtung das äußere Muskelgewebe durchstoßen und das Organ erreicht hat. Im Falle von Tiefensensoren wird dies anhand der Tiefe, bis zu welcher die Eindringeinrichtung in den Organismus eingedrungen ist, erkannt. Hingegen kann bei Verwendung eines magnetischen Positionssensors die aktuelle Position der Eindringeinrichtung innerhalb des Organismus direkt erkannt werden. Geeignete Sensoren wie Kraftsensoren, Ultraschallsensoren und Infrarotsensoren lassen sich zudem vorteilhaft für Zusatzmessungen einsetzen, mit denen festgestellt wird, ob Knochengewebe zwischen der vorgesehenen Injektionsstelle für die Eindringvorrichtung und dem Körpergewebe oder Organ vorhanden ist, welches ein Eindringen der Eindringeinrichtung behindern würde. Gassensoren und insbesondere Luftsensoren ermöglichen die Detektion von Gasen oder Luft in der Eindringeinrichtung bzw. sie Überwachen darin eindringende Luft. Sofern mittels derartiger Gassensoren das Vorhandensein von Luft in der Eindringeinrichtung festgestellt wird, kann die erfindungsgemäße Vorrichtung dazu eingerichtet sein, ein entsprechendes Warnsignal am Signalausgabemittel auszugeben und das Einbringen der Eindringeinrichtung in den Organismus zu blockieren, selbst wenn die zuletzt von der Positionserfassungseinrichtung erfasste Position als geeignet eingestuft wird, um zu vermeiden, dass diese Gase oder die Luft in den Organismus eindringen und dort gesundheitliche Probleme verursachen.

Vorzugsweise umfasst bei der erfindungsgemäßen Vorrichtung die Eindringeinrichtung wenigstens ein Schneidmittel, das zum Aufschneiden des Organismus vorgesehen ist, um der Eindringeinrichtung das Eindringen in den Organismus zu erleichtern. Grundsätzlich sind alle Mittel als Schneidmittel geeignet, die es erlauben, den Organismus lokal aufzuschneiden oder aufzutrennen, um der Eindringeinrichtung ein Eindringen in denselben zu ermöglichen. Dabei kann der Organismus vom Schneidmittel vor dem eigentlichen Eindringen der Eindringeinrichtung aufgeschnitten werden oder während des Eindringens der Eindringeinrichtung. Bei dem Schneidmittel kann es sich insbesondere um eine Schneide oder eine Klinge handeln, die an einem vorderen Endabschnitt der Eindringeinrichtung, mit dem die Eindringeinrichtung zuvorderst in den Organismus eindringt, angeordnet ist, um den Organismus während des Überführens der Eindringeinrichtung in die Endposition direkt vor der Eindringeinrichtung aufzuschneiden und der Eindringeinrichtung damit ein Eindringen in den Organismus zu ermöglichen. Auch ein ausreichend spitz oder keilförmig zulaufend ausgebildeter vorderer Endabschnitt der Eindringeinrichtung, mit dem die Eindringeinrichtung zuvorderst in den Organismus eindringt, kann ein solches Schneidmittel darstellen.

Bei einer vorteilhaften Ausführungsform der erfindungsgemäßen Vorrichtung weist die Eindringeinrichtung wenigstens zwei teleskopartig ineinanderschiebbare und auseinanderziehbare Elemente auf. Grundsätzlich kann die Eindringeinrichtung auch drei oder mehr solcher teleskopartig ineinanderschiebbarer und auseinanderziehbarer Elemente aufweisen. Derartige teleskopartig ineinanderschiebbare und auseinanderziehbare Elemente erlauben es, die Eindringeinrichtung besonders einfach in den Organismus zu injizieren. Besonders bevorzugt ist die Elektrode an oder in einem vorderen Endabschnitt eines dieser Elemente angeordnet, und zwar bei demjenigen Element, das von allen Elementen am weitesten in den Organismus eindringt, was für gewöhnlich beim innersten der ineinandergeschobenen Elemente der Fall ist. Ganz besonders bevorzugt handelt es sich bei dem äußersten dieser Elemente um eine stabil ausgeführte Hülse, die zum Durchstoßen von äußeren Schichten des Organismus wie zum Beispiel der Haut, von Sehnen sowie des äußeren Muskelgewebes vorgesehen ist und die soweit in den Organismus eindringt, bis sie mit einem vorderen Ende die Außenseite eines Organs gerade berührt oder dieser nahe benachbart ist. In dieser Position der Hülse können dann ein oder mehrere teleskopartig in der Hülse aufgenommene Elemente aus der Hülse ausfahren und das Organ unmittelbar kontaktieren oder in das Organ eindringen bzw. eine Außenwand des Organs durchstoßen. Da Organwände der Eindringeinrichtung normalerweise weniger Widerstand entgegensetzen als das Muskelgewebe, können diese teleskopartig in die Hülle bzw. in das äußerste Element geschobenen Elemente weniger stabil oder widerstandsfähig ausgeführt sein als das äußerste Element.

Es sind Ausführungsformen der erfindungsgemäßen Vorrichtung möglich, bei denen die Elektrode wenigstens einen Teilbereich einer Außenfläche der Eindringeinrichtung überdeckt oder bildet und/oder bei denen die Eindringeinrichtung wenigstens ein aufklappbares Mittel umfasst, wobei die Elektrode wenigstens einen Teilbereich einer Außenfläche des aufklappbaren Mittels bedeckt oder bildet, und/oder bei denen die Eindringeinrichtung wenigstens ein expandierbares Mittel umfasst, wobei die Elektrode wenigstens einen Teilbereich einer Außenfläche des expandierbaren Mittels bedeckt oder bildet, und/oder bei denen die Eindringeinrichtung wenigstens ein ausfahrbares Teil umfasst, wobei die Elektrode wenigstens einen Teilbereich einer Außenfläche des ausfahrbaren Teils bedeckt oder bildet. Bevorzugt handelt es sich bei der Elektrode um ein Metallgeflecht, das die entsprechenden Teilbereiche der Außenflächen bildet oder diese bedeckt. Das aufklappbare Mittel ist vorzugsweise in einem zusammengeklappten Zustand in einem Hohlraum der Eindringeinrichtung aufgenommen. Beispielsweise handelt es sich bei dem aufklappbaren Mittel um eine aufklappbare schirmartige Konstruktion wie zum Beispiel um ein Geflecht, das über Federgelenke schirmartig zusammen- und aufklappbar ist. Bei einer Ausführungsform der Erfindung tritt das aufklappbare Mittel aus dem Hohlraum in der Eindringeinrichtung aus, klappt auf und nimmt dabei einen relativ großflächigen aufgeklappten Zustand ein und wird in diesem aufgeklappten Zustand an einer Außenseite eines Organs angelegt, wodurch die den Teilbereich der Außenfläche des aufklappbaren Mittels bedeckende Elektrode mit dem Organ in Kontakt kommt und die Eindringeinrichtung die Endposition eingenommen hat. Bei einer anderen Ausführungsform der Erfindung, bei der die Eindringeinrichtung eine Außenwandung des Organs durchstößt und in eine Kammer des Organs eintritt, tritt das aufklappbare Mittel erst im Inneren der Kammer aus dem Hohlraum aus und klappt auf. Nach Einnehmen des aufgeklappten Zustands fährt die Eindringeinrichtung wieder ein stückweit aus der Kammer hinaus, bis das aufgeklappte aufklappbare Mittel an der Innenwand der Kammer zur Anlage kommt und die den Teilbereich der Außenfläche des aufklappbaren Mittels bedeckende Elektrode nunmehr an der Innenwand der Kammer das Organ kontaktiert. Sobald dies geschieht, hat die Eindringeinrichtung ihre Endposition erreicht. Diesen beiden ein aufklappbares Mittel aufweisenden Ausführungsformen entsprechende Ausführungen der Erfindung sind auch mit einem expandierbaren Mittel anstelle des aufklappbaren Mittels möglich, wobei das expandierbare Mittel entsprechend zunächst in einem zusammengefalteten Zustand in einem Hohlraum der Eindringeinrichtung angeordnet ist, in relativer Nähe zum Organ oder innerhalb einer Kammer des Organs aus dem Hohlraum austritt und expandiert, um anschließend entweder an die Außenseite des Organs oder eine Innenfläche der Kammer des Organs angelegt zu werden, so dass die einen Teilbereich der Außenfläche des expandierbaren Mittels bedeckende Elektrode mit dem Organ in Kontakt kommt. Das expandierbare Mittel kann dabei bekannten Herzballonkathetern ähnlich ausgeführt sein. Zum Expandieren des expandierbaren Mittels ist für die Vorrichtung vorteilhaft eine sich unter anderem durch die Eindringeinrichtung erstreckende Fluidzuführung vorgesehen, welche einer Innenkammer des expandierbaren Mittels ein zum Expandieren notwendiges Fluid zuführt. Bei diesem Fluid kann es sich insbesondere um Wasser oder um Luft handeln. Ferner weist die Vorrichtung vorteilhafterweise ein Fluidreservoir zum Speichern des Fluides oder einen Anschluss zum Verbinden der Vorrichtung mit einem Fluidreservoir auf, über welchen das Fluid der Vorrichtung und letztendlich dem expandierbaren Mittel zugeführt werden kann. Die beschriebenen Ausführungsformen der Erfindung, bei denen das aufklappbare Mittel oder das expandierbare Mittel im Inneren der Kammer des Organs aufgeklappt oder expandiert und anschließend an der Innenwand der Kammer angelegt werden, sind insbesondere deswegen ganz besonders bevorzugt, weil die aufgeklappten und expandierten Mittel wie Widerhaken wirken und die Elektrode bzw. die Eindringeinrichtung dadurch gegen ein versehentliches Herausrutschen aus der Kammer gesichert ist und zudem ein guter Kontakt zwischen Elektrode und Organ besteht. Um die Eindringeinrichtung bzw. die Elektrode wieder aus dem Organismus oder dem Organ zu entfernen, wird das ausklappbare Mittel wieder in den zusammengeklappten Zustand überführt, bzw. dem expandierbaren Mittel wird das Fluid entzogen, wodurch es zusammenfällt, die jeweiligen Mittel werden wieder in den entsprechenden Hohlraum der Eindringeinrichtung eingezogen und die Eindringeinrichtung kann nun bequem aus dem Organ und dem Organismus wieder entfernt werden.

Eine weitere Ausführungsform der vorliegenden Erfindung weist vorteilhaft wenigstens eine Medikamentenzuführung zur Versorgung des Körpergewebes mit wenigstens einem Medikament und wenigstens ein mit der Medikamentenzuführung gekoppeltes Medikamentenreservoir zur Aufnahme von wenigstens einem Medikament und/oder wenigstens einen mit der Medikamentenzuführung gekoppelten Medikamenteneinlass zum Einlassen von wenigstens einem Medikament von außerhalb der Vorrichtung auf. So können beispielsweise ein, zwei, drei oder auch mehr Medikamentenreservoire vorgesehen sein, von denen ein jedes mit einem jeweiligen Medikament gefüllt ist, oder es sind ein, zwei, drei oder mehrere Medikamenteneinlässe vorgesehen, von denen ein jeder zum Einlassen eines jeweiligen Medikaments in die Vorrichtung vorgesehen ist. Ein sich im Medikamentenreservoir befindliches Medikament kann auf pneumatische Weise, beispielsweise durch einen pneumatisch angetriebenen Kolben, aus dem Medikamentenreservoir und in die Medikamentenzuführung gedrückt werden, oder aber es ist wenigstens eine, vorzugsweise steuerbare, Pumpe zur, vorzugsweise gesteuerten, Entnahme des Medikaments aus dem Medikamentenreservoir und zum Einbringen desselben in die Medikamentenzuführung vorgesehen, durch welche es dem Körpergewebe zugeführt wird. Entsprechend kann am Medikamenteneinlass eine bevorzugt steuerbare Pumpe vorgesehen sein, um Medikamente von außerhalb der Vorrichtung in die Medikamentenzuführung zu pumpen. Ferner kann am Medikamenteneinlass ein Anschluss zum Anschließen einer externen Zuführeinrichtung für das Medikament wie zum Beispiel einen Schlauch oder ein externes Medikamentenreservoir vorgesehen sein. Mit diesen Ausführungsformen der vorliegenden Erfindung lassen sich Medikamente wie beispielsweise Adrenalin, Atropin oder Bicarbonate dem Körpergewebe oder einem Organ zuführen. Diese Medikamente werden insbesondere dann gewählt, wenn es sich bei dem Organ um das menschliche Herz handelt. Die Medikamentenzuführung kann eine sich im Inneren der Eindringeinrichtung durch diese erstreckende Zuführleitung aufweisen. Ferner kann die Medikamentenzuführung eine oder mehrere düsenartige Öffnungen aufweisen, mit denen sich ein dem Organ zugeführtes Medikament bevorzugt im Inneren einer Kammer des Organs versprühen lässt. Sofern die Eindringeinrichtung ein expandierbares Teil wie zum Beispiel einen Ballon umfasst, kann dieses beispielsweise eine Membran oder Hülle aus Silikon oder Polyethylen und ein Expansionselement aufweisen, das im expandierten Zustand zumindest auf einer der Innenwand der Kammer des Organs abgewandten Seite zumindest teilweise von der Membran oder Hülle eingehüllt ist. Diese Membran kann mit einer Vielzahl von Öffnungen oder Perforationen versehen sein, durch die ein in einen Zwischenraum zwischen der Membran und dem Expansionselement zugeführtes Medikament aus dem Zwischenraum ins Innere der Kammer des Organs austreten kann, wobei das Medikament wie von einer Vielzahl von Düsen versprüht wird.

Weiterhin ist eine erfindungsgemäße Vorrichtung mit wenigstens einem beweglichen Anlageelement zum Anlegen der Vorrichtung an Körperoberflächen von Organismen bevorzugt. So kann die Vorrichtung beispielsweise ein oder mehrere bewegliche Anlageelemente mit jeweiligen ebenen oder räumlichen Anlageflächen aufweisen, die an einem Gehäuse der Vorrichtung an mehr oder weniger verstreuten Positionen vorgesehen oder einander benachbart angeordnet sind. Desweiteren können auch ein oder mehrere Stützelemente, beispielsweise stiftartige Stützelemente, als Anlageelemente vorgesehen sein, die beliebig geformt sein können und insbesondere mehr oder weniger spitz zulaufend oder auch stumpf mit abgeflachten oder abgerundeten Stirnflächen ausgebildet sein können. Besonders bevorzugt sind die Anlageelemente gegeneinander verschieb- und/oder verkippbar eingerichtet, um sie an Körperoberflächen verschiedenster Formen anpassen zu können und dennoch ein sauberes Anliegen der Vorrichtung an den Organismus zu gewährleisten. Zudem kann für die Vorrichtung eine Klebefixierung an der Körperoberfläche vorgesehen sein, die ein Verrutschen der Vorrichtung unterbindet.

Bei einer Ausführungsform der erfindungsgemäßen Vorrichtung ist eine Tiefe, bis zu der die Eindringeinrichtung in den Organismus eindringt, einstellbar. Beispielsweise kann die Tiefe manuell von einer Bedienperson eingestellt oder vorgegeben werden, wobei die eingestellte Tiefe vorzugsweise nachträglich durch beispielsweise einen Arzt korrigierbar ist. Vorteilhaft weist die Vorrichtung zudem eine Tiefenanzeige auf, wie zum Beispiel einen Bildschirm, an dem die jeweils eingestellte Tiefe und/oder im Betrieb der Vorrichtung die gerade erreichte Tiefe angezeigt wird. Bei anderen Ausführungsformen der erfindungsgemäßen Vorrichtung ist wenigstens ein Tiefenwert fest vorgegeben, bis zu dem die Eindringeinrichtung und/oder die Elektrode in den Organismus eindringt. Derartige Ausführungsformen haben den Vorteil, dass während des Eindringens der Eindringeinrichtung in den Organismus keine Messungen stattfinden müssen und das Eindringen selbst auch nicht gesteuert werden muss, da von vorneherein feststeht, wie tief die Eindringeinrichtung bzw. die Elektrode in den Organismus eindringt. Ein Tiefenwert kann beispielsweise einfach durch die Weite vorgegeben sein, bis zu der die Eindringeinrichtung maximal aus einem Gehäuse der Vorrichtung ausfahren kann. Bei solchen einfachen Ausführungen der Vorrichtung wird die Eindringeinrichtung nach einmaligem Auslösen auf eine voreingestellte Weise in die Endposition überführt. Es können aber auch mehrere unterschiedliche Tiefenwerte vorgegeben werden, die sowohl von der Art des mit Stromimpulsen zu beaufschlagenden Körpergewebes als auch von der Größe des Organismus abhängig sein können. Je nachdem, welches Körpergewebe mit Strom beaufschlagt werden soll und wie groß der Organismus ist, wird ein jeweiliger Tiefenwert, entweder manuell oder automatisch von einer Steuereinheit, ausgewählt und die Eindringeinrichtung dringt bis zu dieser Tiefe in den Organismus ein, ohne dass dieser Vorgang gesteuert werden müsste.

Da bei solchen Vorrichtungen die Tiefe, bis zu der die Eindringeinrichtung in den Organismus eindringt, voreingestellt ist, dringt die Eindringeinrichtung bei allen Organismen gleich tief in dieselben ein. Insbesondere bei lebenden Organismen ist ein solches voreingestelltes Überführen der Eindringeinrichtung in die Endposition für den Organismus jedoch kritisch, da dieses individuelle Unterschiede in der Anatomie von Organismen nicht berücksichtigen kann und die Organismen dabei zu Schaden kommen können. Darüber hinaus kann es zu einem Funktionsausfall der Eindringeinrichtung kommen. Ganz besonders bevorzugt umfasst die erfindungsgemäße Vorrichtung daher wenigstens eine Steuereinheit, die zum Steuern der Abgabe von Stromimpulsen an das Körpergewebe und/oder der Überprüfung der erfassten Position und/oder der Ausgabe des Signals und/oder des Eindringens der Eindringeinrichtung in den Organismus und/oder des Überführens der Eindringeinrichtung in die Endposition und/oder des Kontaktierens des Körpergewebes durch die Elektrode und/oder des Aufklappens des aufklappbaren Mittels und/oder des Expandierens des expandierbaren Mittels und/oder der Zuführung von Medikamenten zum Körpergewebe und/oder des Anpassens einer Positionierung des Anlageelementes an eine äußere Form der Körperoberfläche eingerichtet ist. Eine derartige Steuereinheit erlaubt eine weitgehend automatisierte Arbeitsweise der Vorrichtung. Bei der Steuereinheit kann es sich vorteilhaft um einen Mikroprozessor handeln. Die Steuereinheit kann insbesondere Teil der Positionserfassungseinrichtung und für das Überprüfen der erfassten Position zuständig sein. Insbesondere zum Kontaktieren des Herzens lebender Patienten mit der Elektrode lässt sich das Eindringen der Eindringeinrichtung in den Organismus und das Überführen der Eindringeinrichtung in die Endposition mit einer derartigen Steuereinheit verlässlich und sicher automatisch steuern, ohne dabei auf den Benutzer angewiesen zu sein. Bei anderen Ausführungen der erfindungsgemäßen Vorrichtung ist zu deren Steuerung eine drahtlose Kommunikationsverbindung zwischen der Vorrichtung und einer von dieser beabstandet angeordneten Steuereinheit oder einem Steuergerät vorgesehen, so dass die Überführung der Eindringeinrichtung in die Endposition aber auch beliebig viele weitere und sogar alle anderen Funktionen der Vorrichtung aus der Ferne mittels der entfernten Steuereinheit oder des Steuergerätes bzw. von einem Computer aus gesteuert werden können. Bei der drahtlosen Kommunikationsverbindung kann es sich insbesondere um eine WLAN-Verbindung oder eine Bluetooth-Verbindung handeln.

Vorteilhafterweise weist die erfindungsgemäße Vorrichtung ein Gehäuse auf, wobei die Eindringeinrichtung vom Gehäuse entkoppelbar sein kann. Wenn die Vorrichtung in der Notfallmedizin zur Erstbehandlung von Asystolien eingesetzt wird, sollte die Vorrichtung für gewöhnlich auf dem Patienten verbleiben, bzw. die Eindringeinrichtung sollte nicht vom Gehäuse gelöst werden, bis eine ärztliche Versorgung gewährleistet ist. In Ausnahmefällen kann jedoch eine vom Gehäuse entkoppelbare oder abtrennbare Eindringvorrichtung vorteilhaft sein, da die kleinere Eindringeinrichtung leichter gegen ein Verrutschen aus dem Herzen gesichert werden kann als die im Vergleich dazu relativ große Vorrichtung. Nach erfolgter lokaler Erstversorgung des Patienten kann das Abtrennen der Eindringeinrichtung vom Gehäuse dessen Transport in einem Rettungsfahrzeug zu einem Krankenhaus erleichtern. Die abgekoppelte Eindringeinrichtung kann beispielsweise mit einem Gerät innerhalb des Rettungsfahrzeugs verbindbar sein, welches das Herz des Patienten gegebenenfalls auch während des Transportes mit Stromimpulsen versorgen kann. Im Krankenhaus angekommen kann die Eindringeinrichtung dann von einem Arzt vorsichtig aus dem Herzen und dem Körper des Patienten wieder entfernt werden.

Im Zuge der erfolgreichen Behandlung einer Asystolie mittels einer erfindungsgemäßen Vorrichtung kommt es zu Kontraktionen des Herzmuskels, welche die Gefahr eines Verrutschens der Eindringeinrichtung oder des Austretens der Eindringeinrichtung aus dem Herzen bergen. Hierdurch kann der Kontakt zwischen der Elektrode und dem Herzen und damit die Versorgung des Herzens mit Stromimpulsen unterbrochen werden. Daher ist ganz besonders eine erfindungsgemäße Vorrichtung bevorzugt, die zum Nachführen der Endposition der Eindringeinrichtung eingerichtet ist, wenn das Körpergewebe Bewegungen ausführt. Zum Nachführen der Endposition der Eindringeinrichtung kann die Vorrichtung beispielsweise einen Elektrodenfixierungs- oder Rückzugsmechanismus bzw. ein oder mehrere mechanische, mechatronische, pneumatische oder hydraulische Elemente oder Komponenten bzw. einen Elektrodenfixierungs- oder Rückzugsmechanismus mit wenigstens einem derartigen Element oder einer derartigen Komponente aufweisen. Bei einer zum Nachführen der Endposition der Eindringeinrichtung eingerichteten Vorrichtung ist ein optimaler Kontakt der Elektrode an dem Körpergewebe gewährleistet, selbst wenn das Körpergewebe Bewegungen wie zum Beispiel Kontraktions- oder Expansionsbewegungen ausführt.

Die erfindungsgemäße Vorrichtung lässt sich grundsätzlich autark und kompakt ausführen, so dass sie tragbar und leicht zu handhaben ist. Andererseits kann die erfindungsgemäße Vorrichtung auch Teil eines Systems oder eines multifunktionalen Gerätes sein. Insbesondere wird ein Multifunktionsgerät mit einer erfindungsgemäßen Vorrichtung bevorzugt, das ferner wenigstens einen Defibrillator und/oder wenigstens eine Defibrillatorelektrode und/oder wenigstens einen Herzschrittmacher aufweist. Ein derartiges Multifunktionsgerät vereint vorteilhaft Defibrillatorfunktionen und Schrittmacherfunktionen mit den Funktionen der Vorrichtung der vorliegenden Erfindung. Wird anstelle der derzeit üblichen und zur allgemeinen Verwendung bereitgestellten Defibrillatoren ein derartiges Multifunktionsgerät vorgesehen, so ist es in Notfällen jedermann und sogar auch medizinischen Laien möglich, Asystolien nach erfolglos erfolgter Defibrillation unmittelbar und rasch mittels intrakardialer Schrittmacheraktionen notfallmäßig zu behandeln, und zwar mit ein und demselben Gerät. Hierdurch lässt sich nicht nur die auf Asystolien zurückzuführende Todesrate bei Notfällen enorm senken, auch die im Gefolge von Herzdruckmassagen regelmäßig auftretenden starken Schädigungen von Patienten werden vollständig vermieden.

Die vorliegende Erfindung wird im Folgenden anhand bevorzugter Ausführungsbeispiele unter Zuhilfenahme von Figuren näher beschrieben. Es zeigen:
- Figur 1: einen vereinfachten Querschnitt durch ein Nothilfemodul;
- Figur 2a)-d): die Funktionsweise des Nothilfemoduls der Figur 1;
- Figur 3: einen vereinfachten Querschnitt durch eine Eindringeinrichtung;
- Figur 4a)-c): die Funktionsweise eines Nothilfemoduls mit der Eindringeinrichtung der Figur 3;
- Figur 5a)-c): die Funktionsweise eines Nothilfemoduls mit einer alternativen Eindringeinrichtung;
- Figur 6a)-b): die Funktionsweise eines Nothilfemoduls mit einer weiteren alternativen Eindringeinrichtung;
- Figur 7: einen Querschnitt durch den Ballon des Nothilfemoduls der Figuren 6a)-b);
- Figur 8a)- b): die Funktionsweise eines weiteren Nothilfemoduls;
- Figur 9: ein Multifunktionsnothilfegerät mit einem Nothilfemodul;
- Figur 10a-b): Nachführungseinrichtungen für Eindringeinrichtungen;
- Figur 11a-b): Nachführungseinrichtungen mit unterschiedlichen Federn.

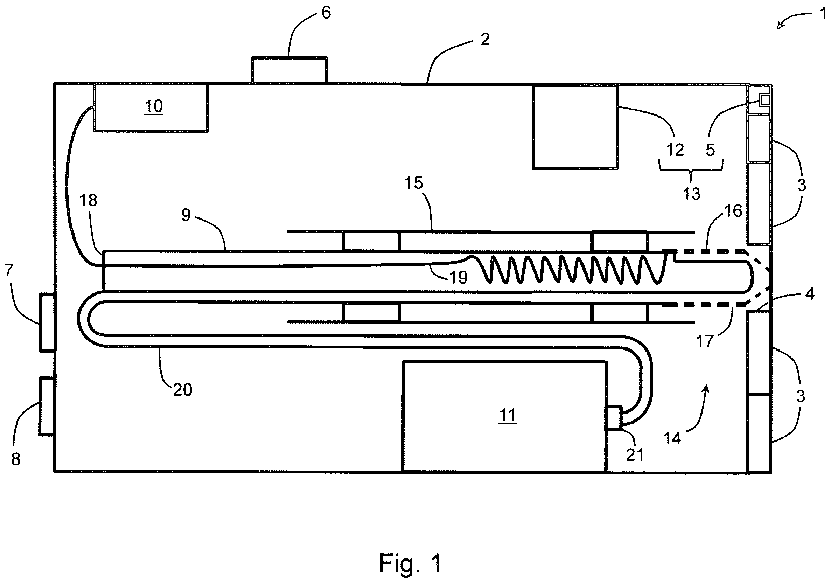

In der Figur 1 ist ein stark vereinfachter, nicht maßstabsgetreuer und wegen der besseren Darstellbarkeit stark schematischer Querschnitt durch ein Nothilfemodul 1 zur Beaufschlagung des Herzens menschlicher Patienten mit Stromimpulsen dargestellt, das einer grundlegenden Ausführungsform der erfindungsgemäßen Vorrichtung entspricht.

Das Nothilfemodul 1 weist ein zylinderförmiges Gehäuse 2 auf, dessen kreisförmige Grundfläche von einer Mehrzahl relativ zueinander sowie relativ zum Gehäuse 2 verschiebbarer Anlageelemente 3 gebildet wird, wobei die Grundfläche eine mittig ausgesparte Öffnung 4 aufweist und eine Sensoranordnung 5, bei der es sich um eine Kombination aus einem Infrarotsensor und einem Ultraschallsensor handelt, in die Grundfläche eingelassen ist. An der seitlichen Mantelfläche des Gehäuses 2 ist eine manuell bedienbare Aktivierungstaste 6 angeordnet, während in der im Wesentlichen flach ausgebildeten Deckfläche des Gehäuses 2 eine rote Leuchtdiode 7 und eine grüne Leuchtdiode 8 angeordnet sind. Desweiteren umfasst das Nothilfemodul 1 eine längliche, durchgängig hohle Eindringeinrichtung 9, eine Batterie 10, ein Medikamentenreservoir 11 und eine Steuereinheit 12 in Form eines Mikroprozessors, die alle im Inneren des Gehäuses 2 aufgenommen sind. Dabei ist die Steuereinheit 12 mit der Sensoranordnung 5 zum Zwecke des Datenaustausches verbunden und bildet mit dieser zusammen eine Positionserfassungseinrichtung 13. Aus Gründen der Übersichtlichkeit ist die Verbindung zwischen der Steuereinheit 12 und der Sensoranordnung 5 in der Figur 1 jedoch nicht dargestellt. Ebenso sind Verbindungen der Steuereinheit 12 mit anderen Bauteilen des Nothilfemoduls 1, die wie im Folgenden ausgeführt wird von der Steuereinheit 12 angesteuert werden, aus denselben Gründen in der Figur 1 nicht eingezeichnet.

Die im Wesentlichen hohle Eindringeinrichtung 9, bei der es sich um eine Metallkanüle handelt, erstreckt sich mittig entlang der Längsachse des Gehäuses 2, wobei ein vorderer Endabschnitt 14 der Eindringeinrichtung 9 mit einem im Wesentlichen spitz zulaufenden Ende der Öffnung 4 zugewandt ist und mit dieser fluchtet bzw. in die Öffnung 4 ragt, ohne die Öffnung 4 jedoch zu durchstoßen. Eine Mechanik 15 ist zum Verschieben der Eindringeinrichtung 9 entlang der Längsachse des Gehäuses 2 vorgesehen. Bis zu einem gewissen Grad kann die Mechanik 15 die Eindringeinrichtung 9 auch quer zu dieser Längsachse verkippen, so dass die jeweiligen Längsachsen des Gehäuses 2 und der verkippten Eindringeinrichtung 9 einen Winkel einschließen. Mittels der Mechanik 15 lässt sich die Eindringeinrichtung 9 mit dem vorderen Endabschnitt 14 voran durch die Öffnung 4 hindurch aus dem Gehäuse 2 schieben. Die Eindringeinrichtung 9 ist am vorderen Endabschnitt 14 mit einem Metallgeflecht überzogen, welches als Elektrode 16 vorgesehen ist. Ferner ist der vordere Endabschnitt 14 perforiert und weist eine Mehrzahl an Durchlässen 17 auf, die sich sowohl durch die Wandung der Eindringeinrichtung 9 als auch durch die Elektrode 16 erstrecken. Eine sich im Inneren der Eindringeinrichtung 9 durch die Eindringeinrichtung 9 erstreckende, abschnittsweise gewickelte und aus einem hinteren Ende 18 der Eindringeinrichtung 9 aus dieser austretende elektrische Leitung 19 verbindet die Elektrode 16 mit der Batterie 10, wodurch die Elektrode 16 von der Batterie 10 mit Stromimpulsen versorgt werden kann. Desweiteren erstreckt sich eine an einer Innenfläche der Eindringeinrichtung 9 anliegende schlauchförmige Fluidleitung 20 im Inneren der Eindringeinrichtung 9 durch die Eindringeinrichtung 9, die wie die elektrische Leitung 19 am hinteren Ende 18 der Eindringeinrichtung 9 aus dieser austritt und über eine Pumpe 21 mit dem mit einem flüssigen Medikament gefüllten Medikamentenreservoir 11 verbunden ist. Dabei weist die Fluidleitung 20 außerhalb der Eindringeinrichtung 9 eine ausreichende Länge und Flexibilität auf, um ein Austreten der Eindringeinrichtung 9 aus der Öffnung 4 zu erlauben, ohne dass diese durch die Verbindung der Fluidleitung 20 mit der Pumpe 21 behindert würde. Im vorderen Endabschnitt 14 der Eindringeinrichtung 9 mündet die Fluidleitung 20 in einen im Inneren der Eindringeinrichtung 9 die Durchlässe 17 der Perforation überdeckend ausgebildeten und mit den Durchlässen 17 kommunizierenden Zwischenraum, der infolge der durchgängigen Durchlässe 17 zur Außenseite der Eindringeinrichtung 9 hin geöffnet ist.

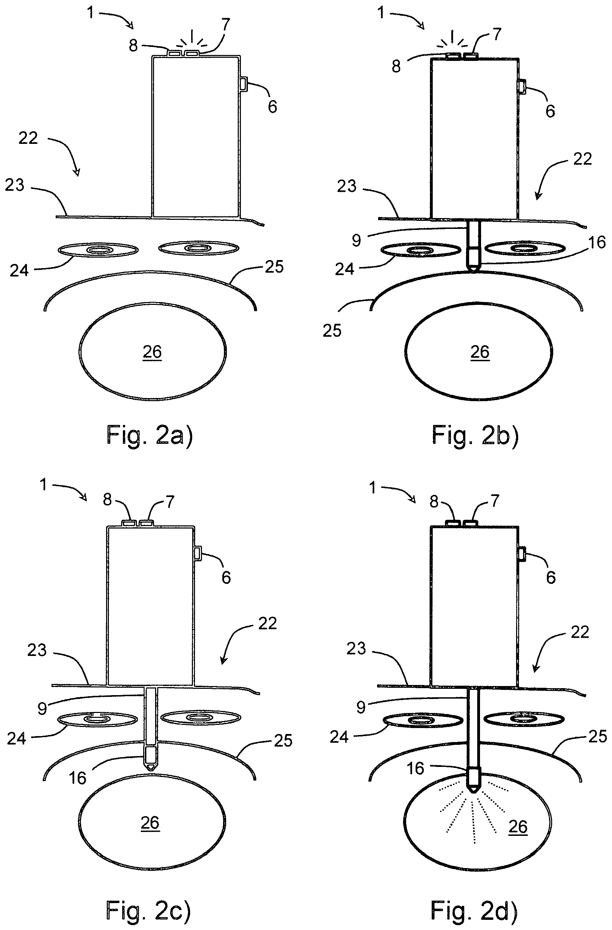

Im Folgenden wird nun die Funktionsweise des Nothilfemoduls 1 anhand der notfallmäßigen Erstbehandlung einer Asystolie unter Bezugnahme auf die Figuren 2a)-d) erläutert. In den Figuren 2a)-d) ist ausschnittsweise ein Querschnitt durch einen Teil des Körpers 22 bzw. einen Teil des linken Brustbeins eines rücklings liegenden Patienten mit Teilen der Haut 23, der Rippen 24 und einem Teil des Herzens 25 einschließlich einer Herzkammer 26 zu sehen. Vorliegend handelt es sich bei den Rippen 24 genauer um die dritte und die vierte Rippe und bei der Herzkammer 26 um die rechte Herzkammer oder Ventrikel.

Von einem Ersthelfer wird das Nothilfemodul 1 zunächst im Bereich des linken Brustbeins in ungefährer Nähe zum Herzen 25 an den Körper 22 angelegt und mittels der Aktivierungstaste 6 eingeschaltet. Figur 2a) zeigt das am Körper 22 anliegende Nothilfemodul 1, das mit der Grundfläche des Gehäuses 2 auf dem Körper 22 aufliegt. Von der Sensoranordnung 5 der Positionserfassungseinrichtung 13, bzw. von deren Infrarotsensor, wird nach Einschalten des Nothilfemoduls 1 die Position der Öffnung 4 in der Grundfläche des Gehäuses 2, bei manchen Ausführungsformen des Nothilfemoduls 1 die Position der Elektrode 16, in Bezug auf die rechte Herzkammer 26 erfasst. Ferner wird von der Sensoranordnung 5 das Vorhandensein einer Rippe 24 zwischen der Öffnung 4 bzw. der Elektrode 16 und der Herzkammer 26 detektiert. Die von der Sensoranordnung 5 erfassten Daten werden von diesem an die Steuereinheit 12 der Positionserfassungseinrichtung 13 übergeben und von dieser daraufhin überprüft, ob die erfasste gegenwärtige Position des Nothilfemoduls 1 auf dem Körper 22 bzw. der Öffnung 4 oder der Elektrode 16 in Bezug auf die Herzkammer 26 zum Einbringen der Eindringeinrichtung 9 in den Körper 22 und zum Überführen der Eindringeinrichtung 9 in eine Endposition geeignet ist, in welcher die Elektrode 16 das Herz 25 kontaktiert. In der in der Figur 2a) gezeigten Stellung des Nothilfemoduls 1 befindet sich die Öffnung 4 zwar oberhalb des Herzens 25 und auch oberhalb der Herzkammer 26, der Weg zum Herzen 25 wird der Eindringeinrichtung 9 aber durch eine der Rippen 24 versperrt. Dies wird von der Steuereinheit 12 erkannt, die infolgedessen die rote Leuchtdiode 7 dazu veranlasst, aufzuleuchten und dem Ersthelfer damit zu signalisieren, dass ein Überführen der Eindringeinrichtung in die Endposition in der derzeitigen Stellung des Nothilfemoduls 1 bzw. bei der derzeitigen Position der Öffnung 4 oder der Elektrode 16 in Bezug auf das Herz 25 oder die Herzkammer 26 nicht möglich ist. Der Ersthelfer weiß somit, dass er das Nothilfemodul 1 an eine andere Stelle versetzen muss, woraufhin auf die soeben beschriebene Weise wiederum von der Sensoranordnung 5 die Position der Öffnung 4 bzw. der Elektrode 16 in Bezug auf das Herz 25 oder die Herzkammer erfasst und von der Positionserfassungseinrichtung 13 daraufhin überprüft wird, ob die Eindringeinrichtung 9 nunmehr in die gewünschte Endposition überführt werden kann oder nicht. Ist dies nicht der Fall, leuchtet die rote Leuchtdiode 7 abermals auf, sofern sie zuvor erloschen ist, oder sie fährt fort zu leuchten, und der Vorgang wird so lange wiederholt, bis eine geeignete Stellung für das Nothilfemodul 1 bzw. eine geeignete Position der Öffnung 4 oder der Elektrode 16 in Bezug auf das Herz 25 oder die Herzkammer 26 gefunden ist.

Figur 2b) zeigt das Nothilfemodul 1 nach dessen Versetzen aus der in der Figur 2a) gezeigten ungeeigneten Stellung nunmehr in einer zum Einbringen der Eindringeinrichtung 9 in die Endposition geeigneten Stellung. Die Öffnung 4 befindet sich zwar immer noch direkt oberhalb des Herzens 25 und der Herzkammer 26, jedoch ist der Eindringeinrichtung 9 der Weg zum Herzen 25 nicht mehr durch die Rippen 24 versperrt. Infolge der beweglichen Anlageelemente 3, die sich bei einer Ausführungsform entweder aufgrund einer geeigneten Aufhängung von alleine verschieben oder die bei einer anderen Ausführungsform von der Steuereinheit 12 entsprechend verschoben werden, passt sich die Grundfläche des Gehäuses 2 an die spezifische Form des Körpers 22 an dieser Stelle an, wodurch das Nothilfemodul 1 in sichere Anlage zum Körper 22 kommt, wobei das Nothilfemodul 1 durch ein zwischen den Anlageelementen 3 und der Haut 23 des Körpers 22 vorgesehenes Haftmittel gegen ein eventuelles Verrutschen zusätzlich gesichert ist. Anhand der von der Sensoranordnung 5 gelieferten Daten erkennt die Steuereinheit 12 die Eignung der aktuellen Stellung des Nothilfemoduls 1 bzw. der Position der Öffnung 4 oder der Elektrode 16 in Bezug auf das Herz 25 oder die Herzkammer 26 zum Überführen der Eindringeinrichtung 9 in die Endposition, weshalb sie die grüne Leuchtdiode 8 veranlasst, anstatt der roten Leuchtdiode 7 aufzuleuchten. Für den Ersthelfer ist dies das Signal, das Einbringen der Eindringeinrichtung 9 in den Körper 22 bzw. das Überführen der Eindringeinrichtung 9 in die Endposition einzuleiten.

Hierzu betätigt er die Aktivierungstaste 6. Nun steuert die Steuereinheit 12 die Mechanik 15 an, woraufhin diese die Eindringeinrichtung 9 mit dem vorderen Endabschnitt 14 voran durch die Öffnung 4 schiebt und diese in den Körper 22 und durch die Haut 23 sowie zwischen den Rippen 24 hindurch drückt. Da der vordere Endabschnitt 14 ein spitz zulaufendes Ende hat, das sowohl die Haut 23 als auch darunterliegendes Muskelgewebe und Knorpel während des Eindringens des vorderen Endabschnitts 14 in den Körper 22 leicht zerteilt, wird der Eindringeinrichtung 9 das Eindringen in den Körper 22 erleichtert. Weil zudem die Fluidleitung 20 flexibel ist und eine ausreichende Länge aufweist und weil ferner die elektrische Leitung 19 abschnittsweise aufgewickelt ist und sich während des Ausfahrens der Eindringeinrichtung 9 auf eine ausreichende Länge abwickeln lässt, wird das Ausfahren der Eindringeinrichtung 9 aus dem Gehäuse 2 weder durch die Fluidleitung 20 noch durch die elektrische Leitung 19 behindert. Dabei wird der Vorgang des Eindringens des vorderen Endabschnitts 14 in den Körper 22 von der Positionserfassungseinrichtung 13 mittels der Sensoranordnung 5 permanent überwacht, wobei die Positionserfassungseinrichtung 13 mittels der Steuereinheit 12, welche die Mechanik 15 entsprechend ansteuert, gegebenenfalls regelnd in den Eindringvorgang eingreift. Während des gesamten Eindringvorganges wird vom Ultraschallsensor der Sensoranordnung 5 die Tiefe gemessen, bis zu welcher der vordere Endabschnitt 14 in den Körper 22 jeweils eingedrungen ist.

In der Figur 2b) ist die Eindringeinrichtung 9 bereits so weit in den Körper 22 eingedrungen, dass ein vorderes Ende der Eindringeinrichtung 9 eine Kammerscheidewand des Herzens 25 gerade berührt bzw. gerade an die Kammerscheidewand des Herzens 25 anstößt. Grundsätzlich ist eine Beaufschlagung des Herzens 25 mit Stromimpulsen bereits jetzt möglich, sofern die Elektrode 16 auch das spitze Ende der Eindringeinrichtung 9 überdeckt. Bei manchen Ausführungsformen bzw. Betriebsweisen des Nothilfemoduls 1 stellt die in der Figur 2b) gezeigte Position der Eindringeinrichtung 9 daher bereits deren Endposition dar. Im vorliegenden Fall, in dem mit dem Nothilfemodul 1 eine Asystolie behandelt werden soll, ist ein äußerliches Berühren des Herzens 25 durch die Elektrode 16 für den angestrebten Behandlungszweck jedoch nicht ausreichend, da möglicherweise nach einer ersten Herzkontraktion, welche durch von der Elektrode 16 abgegebene Stromimpulse ausgelöst wird, die Position bzw. Ausdehnung des Herzens 25 verändert wird, so dass die von der Steuereinheit 12 gesteuerte Mechanik 15 mit dem Vorantreiben der Eindringeinrichtung 9 fortfährt und die Elektrode 16 in das Herz 25 hinein bzw. in dessen Kammerscheidewand eindringen lässt, was in der Figur 2c) zu sehen ist. Auch die in der Figur 2c) gezeigte Position der Eindringeinrichtung 9 kann für einige Ausführungsformen oder Betriebsweisen des Nothilfemoduls 1 deren Endposition darstellen, da es der Elektrode 16 ermöglicht ist, Stromimpulse direkt in die Kammerscheidewand abzugeben.

Im Falle des gezeigten Nothilfemoduls 1 wird die Eindringeinrichtung 9 jedoch noch ein Stück weiter vorangetrieben, bis deren vorderer Endabschnitt 14 mit der Elektrode 16 wie in der Figur 2d) gezeigt teilweise in die Herzkammer 26 ragt und teilweise in der Kammerscheidewand steckt. Für die vorliegende Ausführung des Nothilfemoduls 1 entspricht dies der Endposition der Eindringeinrichtung 9. Sobald sich die Eindringeinrichtung 9 in der in der Figur 2d) dargestellten Endposition befindet, sorgt die Steuereinheit 12 dafür, dass von der Batterie 10 Stromimpulse über die elektrische Leitung 19 und die Elektrode 16 an das Herz 25 abgegeben werden. Ferner steuert die Steuereinheit 12 die Pumpe 21 an, um das im Medikamentenreservoir 11 befindliche Medikament in die Fluidleitung 20 und durch diese hindurch bis in den Zwischenraum im vorderen Endabschnitt 14 der Eindringeinrichtung 9 zu pumpen, wo es durch die Durchlässe 17 hindurchtritt und in der Herzkammer 26 verspritzt wird. Nach erfolgter Behandlung der Asystolie wird die Eindringeinrichtung 9 vom Rest des Nothilfemoduls 1 entkoppelt und vollständig vom Gehäuse 2 getrennt. Der Patient kann nun in ein Krankenhaus gebracht werden, wo schließlich auch die Eindringeinrichtung 9 aus dessen Körper 22 entfernt wird.

Sollte die Behandlung der Asystolie wieder Erwarten erfolglos bleiben, so kann das Nothilfemodul 1 dank seiner flachen Deckfläche auch zur Anwendung von Herzdruckmassagen verwendet werden. Dabei kann die Eindringeinrichtung 9 das Herz 25 auch während der Herzdruckmassage als begleitende Maßnahme mit Strom versorgen.

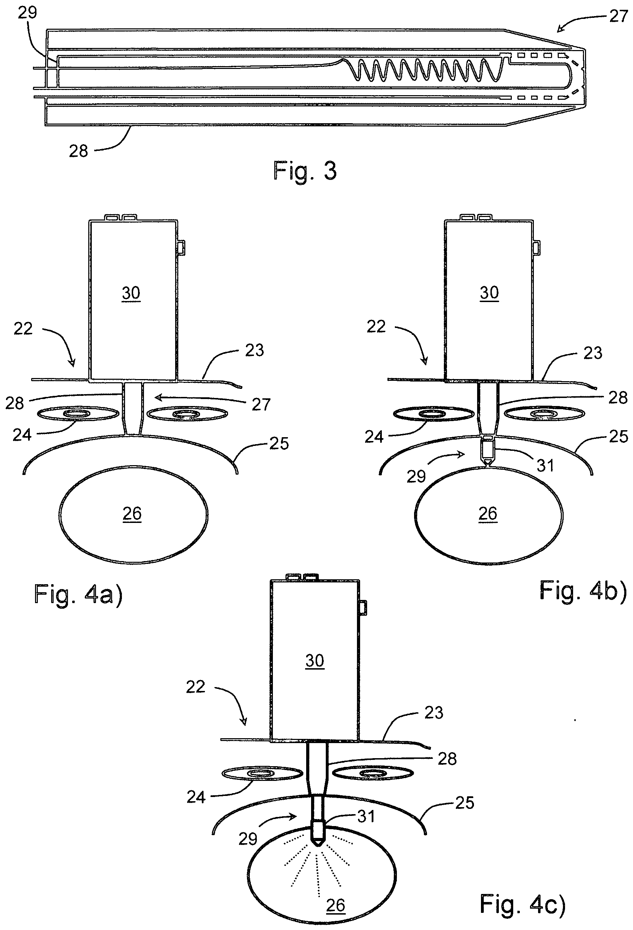

Einen vereinfachten Querschnitt durch eine zur Eindringeinrichtung 9 alternativ ausgeführte Eindringeinrichtung 27 zeigt die Figur 3. Die Eindringeinrichtung 27 umfasst ein äußeres Hülsenelement 28 und ein inneres Elektrodenelement 29, wobei das Elektrodenelement 29 im Hülsenelement 28 aufgenommen und teleskopartig aus diesem ausziehbar ist. Während der Aufbau des Elektrodenelements 29 im Wesentlichen demjenigen der Eindringeinrichtung 9 des Nothilfemodules 1 entspricht und daher nicht näher erläutert wird, handelt es sich beim Hülsenelement 28 im Wesentlichen um eine stabile Hülse mit einem sich verjüngenden vorderen Endabschnitt, die zum Schutz des Elektrodenelements 29 während des Einbringens der Eindringeinrichtung 27 in den Körper 22 vorgesehen ist. Erst wenn beim Einbringen der Eindringeinrichtung 27 in den Körper 22 die größten Widerstände überwunden worden sind, fährt das Elektrodenelement 29 aus dem Hülsenelement 28 aus und dringt ohne das Hülsenelement 28 in das Herz 25 ein.

In den Figuren 4a)-c) ist dies anhand eines Nothilfemoduls 30 näher dargestellt, das sich vom Nothilfemodul 1 vor allem darin unterscheidet, dass es anstatt der Eindringeinrichtung 9 die Eindringeinrichtung 27 der Figur 3 aufweist.

Wenn im Betrieb des Nothilfemoduls 30 die Eindringeinrichtung 27 in den Körper 22 eindringt um ihre Endposition einzunehmen, werden die schwer zu durchdringenden äußeren Bereiche des Körpers 22 wie die Haut 23, Muskelgewebe und Knorpel zunächst von dem stabilen Hülsenelement 28 auf dem Weg bis hin zur Kammerscheidewand des Herzens 25 durchdrungen. Während des Eindringens in den Körper 22 bleibt das Elektrodenelement 29 und insbesondere das in der Figur 4a) nicht sichtbare an dessen vorderem Endabschnitt als Elektrode 31 angeordnete Metallgeflecht vom Hülsenelement 28 geschützt. Figur 4a) zeigt die Situation, in der das Hülsenelement 28 so weit in den Körper 22 eingedrungen ist, dass dessen vorderes Ende die Kammerscheidewand des Herzens 25 berührt. Erst wenn wie in der Figur 4a) dargestellt die äußeren widerstandsfähigen Körperschichten von der Eindringeinrichtung 27 bzw. dem Hülsenelement 28 durchdrungen worden sind, wird das Elektrodenelement 29 aus dem Hülsenelement 28 ausgefahren und kann nun mit seinem vorderen Endabschnitt und der daran angeordneten Elektrode 31 relativ ungehindert in das Herz 25 eindringen, das dem Elektrodenelement 29 einen vergleichsweise geringen Widerstand entgegensetzt. In der Figur 4b) ist das Elektrodenelement 29 bereits so weit in das Herz 25 eingedrungen, dass die Elektrode 31 in dessen Kammerscheidewand steckt. Bei einer ersten Betriebsweise des Nothilfemoduls 30 entspricht der in der Figur 4b) gezeigte Zustand der Eindringeinrichtung 27 bereits deren Endposition, da die Elektrode 31 das Herz 25 kontaktiert und dadurch in die Lage versetzt ist, das Herz 25 mit Stromimpulsen zu versorgen. Bei einer zweiten Betriebsweise des Nothilfemoduls 30 wird das Elektrodenelement 29 so weit in das Herz 25 gedrückt, bis der vordere Endabschnitt des Elektrodenelements 29 mit der Elektrode 31 teilweise in die Herzkammer 26 ragt, während die Elektrode 31 gleichzeitig teilweise in der Kammerscheidewand steckt. In der zweiten Betriebsweise des Nothilfemoduls 30 entspricht nun dieser in der Figur 4c) gezeigte Zustand der Endposition der Eindringeinrichtung 27. Über den in der Kammerscheidewand steckenden Teil der Elektrode 31 kann das Herz 25 nun mit Stromimpulsen beaufschlagt werden während gleichzeitig aus dem in die Herzkammer 26 ragenden Teil des Elektrodenelements 29 zur medikamentösen Behandlung des Herzens 25 Medikamente in die Herzkammer 26 versprüht werden können.

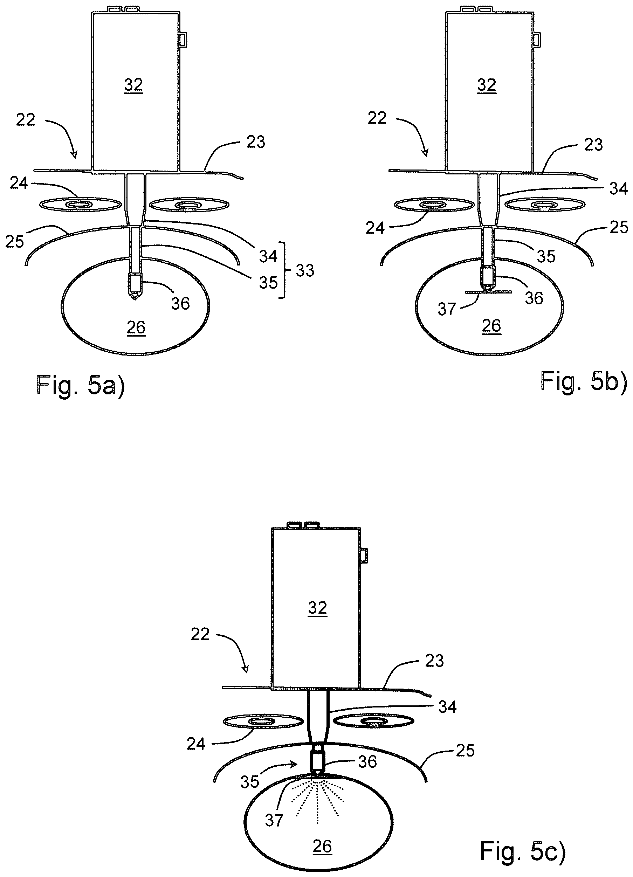

Die Funktionsweise eines weiteren Nothilfemoduls 32 mit einer anderen Eindringeinrichtung 33 ist in den Figuren 5a)-c) zu sehen. Die Eindringeinrichtung 33 umfasst ähnlich der Eindringeinrichtung 27 der Figur 3 ein Hülsenelement 34 und ein in dem Hülsenelement 34 teleskopartig aufgenommenes und aus diesem ausfahrbares Elektrodenelement 35 mit einem an dessen vorderem Endabschnitt als Elektrode 36 angeordneten Metallgeflecht. Das Elektrodenelement 35 umfasst zusätzlich zur Elektrode 36 eine in einem zugeklappten Zustand in dessen Innerem aufgenommene und in den Figuren 5b) und 5c) zu sehende aufklappbare Klappelektrode 37, die nach Austreten aus dem Inneren des Elektrodenelements 35 schirmartig aufklappen und einen aufgeklappten Zustand einnehmen kann. Bei der Klappelektrode 37 handelt es sich im Wesentlichen um ein Metallgeflecht mit einem Klappmechanismus ähnlich demjenigen eines Regen- oder Sonnenschirms, wobei das Metallgeflecht im aufgeklappten Zustand der Klappelektrode 37 zwischen einzelnen Streben des Klappmechanismus aufgespannt ist.

Wie beim Nothilfemodul 30 in den Figuren 4a)-c) wird auch beim Nothilfemodul 32 zum Einbringen der Eindringeinrichtung 33 in den Körper 22 zunächst das Hülsenelement 34 bis zur Kammerscheidewand des Herzens 25 in den Körper 22 vorangetrieben, wobei das Elektrodenelement 35 während des Einführens des Hülsenelements 34 in den Körper 22 zu dessen Schutz im Hülsenelement 34 aufgenommen ist. Sodann wird das Elektrodenelement 35 aus dem Hülsenelement 34 ausgefahren. Das Elektrodenelement 35 durchstößt die Kammerscheidewand des Herzens 25, bis ein Teil davon wie in der Figur 5a) gezeigt in die Herzkammer 26 ragt. Daraufhin tritt die Klappelektrode 37 aus dem Inneren des Elektrodenelements 35 aus und nimmt ihren aufgeklappten Zustand ein, in welchem die Klappelektrode eine vergleichsweise große Fläche aufweist, was in der Figur 5b) zu sehen ist. In den Figuren 5a) und 5b) befindet sich die Elektrode 36 zur besseren Verdeutlichung der Funktionsweise des Nothilfemoduls 32 zwar vollständig im Inneren der Herzkammer 26, in den meisten Fällen steckt sie in den beschriebenen Situationen jedoch teilweise in der Kammerscheidewand. Nach dem Aufklappen der Klappelektrode 37 wird das Elektrodenelement 35 wieder ein Stück weit in das Hülsenelement 34 zurückgezogen, bis die aufgeklappte Klappelektrode 37 an der Innenwand der Herzkammer 26 anliegt. Nunmehr befindet sich die Elektrode 36 wie aus Figur 5c) ersichtlich im Inneren der Kammerscheidewand, während die Klappelektrode 37 über die Innenwand der Herzkammer 26 mit dem Herzen 25 in Kontakt ist. Der in der Figur 5c) gezeigte Zustand der Eindringeinrichtung 33, bzw. des Hülsenelements 34 und des Elektrodenelements 35 einschließlich der Klappelektrode 37, stellt für die Eindringeinrichtung 33 des Nothilfemoduls 32 die Endposition dar. Hierbei wirkt die aufgeklappte Klappelektrode 37 vorteilhaft ähnlich einem Widerhaken, der ein versehentliches Verrutschen oder Herausziehen des Elektrodenelements 35 aus dem Herzen 25 während einer durch Stromimpulse ausgelösten Herzaktion verhindert. Eine Beaufschlagung des Herzens 25 mit Stromimpulsen ist in dieser Endposition der Eindringeinrichtung 33 durch Abgabe von Stromimpulsen über die Elektrode 36 an die Kammerscheidewand als auch über die Klappelektrode 37 an die Innenwand der Herzkammer 26 möglich. Dabei können verschiedene oder gleiche Stromimpulse von der Elektrode 36 und der Klappelektrode 37 simultan oder zeitversetzt an das Herz 25 abgegeben werden, oder es werden situationsbedingt Stromimpulse von nur einer der beiden Elektroden 36 oder 37 an das Herz 25 abgegeben. Gleichzeitig können über das vordere Ende der Eindringeinrichtung 33 Medikamente in die Herzkammer 26 eingebracht werden.

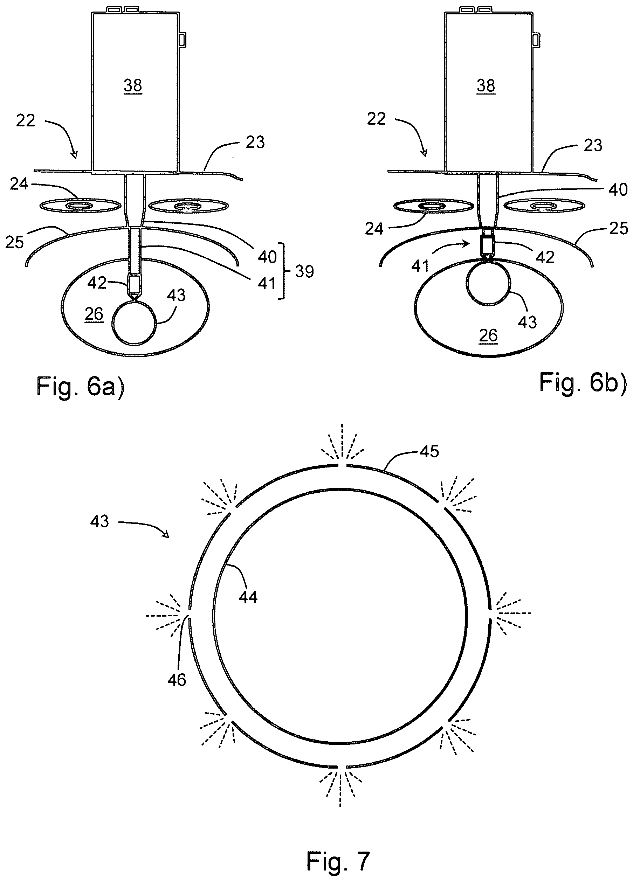

Bei einer weiteren Ausführungsform der Eindringeinrichtung ist die Klappelektrode 37 durch eine Art expandierbarer oder aufblasbarer Elektrode ersetzt. In den Figuren 6a) und 6b) ist die Funktionsweise eines Nothilfemoduls 38 mit einer derartigen Eindringeinrichtung 39 dargestellt. Auch die Eindringeinrichtung 39 weist ein Hülsenelement 40 und ein in das Hülsenelement 40 teleskopartig eingezogenes und in diesem aufgenommenes Elektrodenelement 41 auf, dessen vorderer Endabschnitt umfänglich mit einem Metallgeflecht als Elektrode 42 versehen ist. Das Elektrodenelement 41 weist ferner einen in seinem Inneren in einem zusammengefalteten Zustand aufgenommenen Ballon 43 auf, dessen äußere Oberfläche wie der vordere Endabschnitt des Elektrodenelements 41 auch mit einem Metallgeflecht überzogen ist. Der Ballon 43 kann über ein in den Figuren 6a) und 6b) nicht sichtbares, im Nothilfemodul 38 angeordnetes Wasserreservoir mit Wasser gefüllt und dadurch expandiert werden, wodurch er am vorderen Ende des Elektrodenelements 41 aus dessen Innerem austritt.

Ähnlich der Eindringeinrichtung 33 des Nothilfemoduls 32 dringt auch bei der Eindringeinrichtung 39 des Nothilfemoduls 38 zunächst deren Hülsenelement 40 bis zur Kammerscheidewand des Herzens 25 in den Körper 22 ein, woraufhin das Elektrodenelement 41 aus dem Hülsenelement 40 ausfährt und die Kammerscheidewand durchstößt, bis es wie das Hülsenelement 34 der Eindringeinrichtung 33 in der Figur 5a) mit einem vorderen Teilabschnitt in die Herzkammer 26 ragt. Hierauf wird Wasser vom Wasserreservoir in den zusammengefalteten und im Inneren des Elektrodenelementes 41 aufgenommenen Ballon 43 gepumpt, woraufhin dieser expandiert und dabei aus dem Inneren des Elektrodenelementes 41 austritt. Figur 6a) zeigt die Situation unmittelbar nach dem Befüllen des Ballons 43, der vollständig im Inneren der Herzkammer 26 aufgenommen ist. Alsdann wird das Elektrodenelement 41 wieder ein Stück weit in das Hülsenelement 40 eingezogen, bis die Eindringeinrichtung 39 wie in der Figur 6b) gezeigt ihre Endposition erreicht hat, bei welcher der expandierte Ballon 43 an der Innenwand der Herzkammer 26 anliegt und dadurch als mechanische Sicherung gegen ein Verrutschen des Elektrodenelements 41 oder ein versehentliches Herausziehen des Elektrodenelements 41 aus dem Herzen 25 wirkt. Nunmehr besteht wie im Falle des Nothilfemoduls 32 ein elektrischer Kontakt zwischen der sich im Inneren der Kammerscheidewand befindlichen Elektrode 42 und der Kammerscheidewand einerseits und dem Ballon 43 und der Innenwand der Herzkammer 26 andererseits, so dass ebenso wie im Zusammenhang mit der Figur 5c) beschrieben gleiche oder verschiedene Stromimpulse aufeinanderfolgend, simultan oder einzeln mittels der Elektrode 42 und dem Ballon 43 an das Herz 25 abgegeben werden können.

Einen vergrößerten Querschnitt durch den Ballon 43 zeigt die Figur 7. Wie in der Figur 7 zu sehen ist, umfasst der Ballon 43 im Wesentlichen ein Expansionselement 44, das von einer Membran 45 eingehüllt ist, an deren Außenfläche das als Elektrode wirkende Metallgeflecht angeordnet ist. Die aus Silikon oder Polyethylen gefertigte Membran 45 ist mit einer Vielzahl gleichmäßig über deren Oberfläche verteilter Öffnungen 46 versehen. Zum Befüllen des Ballons 43 wird das Wasser ins Innere des Expansionselementes 44 eingeleitet, woraufhin dieses anschwillt und sein Volumen vergrößert. Nach dem Befüllen des Ballons 43 bzw. dem Expandieren des Expansionselementes 44 wird nunmehr ein Medikament zwischen das Expansionselement 44 und die Membran 45 eingelassen, welches sich um das Expansionselement 44 herum verteilt und aus den Öffnungen 46 austritt, wodurch das Medikament gleichmäßig im Inneren der Herzkammer 26 verspritzt wird.

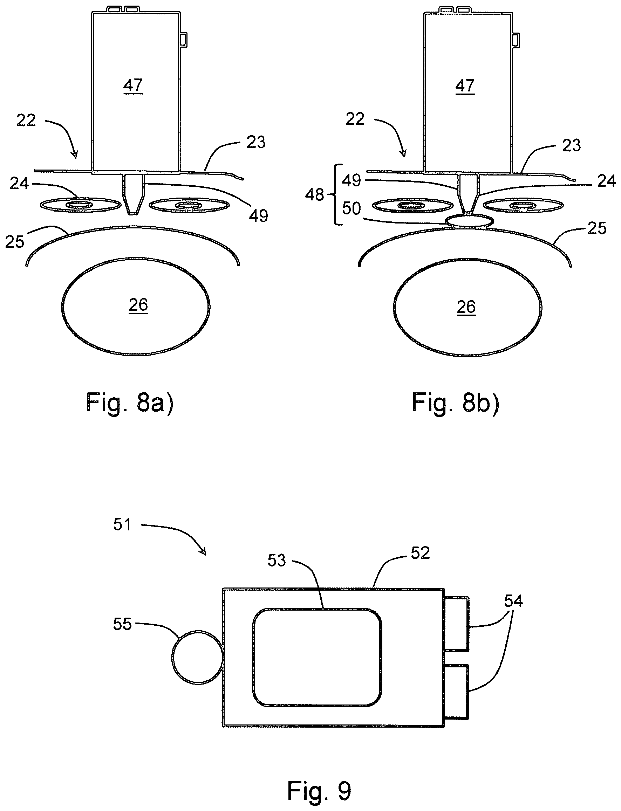

Schließlich ist in den Figuren 8a) und 8b) die Funktionsweise eines weiteren Nothilfemoduls 47 dargestellt. Das Nothilfemodul 47 weist eine Eindringeinrichtung 48 auf, die ein längliches Hülsenelement 49 ähnlich den zuvor beschriebenen Hülsenelementen und einen dem zuvor beschriebenen Expansionselement 44 ähnlichen Ballon 50 aufweist, jedoch fehlt bei der Eindringeinrichtung 48 ein Elektrodenelement, wie es bei den vorherigen Beispielen beschrieben wurde. Der Ballon 50 ist in einem zusammengefalteten, nicht expandierten Zustand im Inneren des Hülsenelements 49 aufgenommen. Er ist mittels Luft aufblasbar, die aus einem entsprechenden Behälter im Gehäuse des Nothilfemoduls 47 stammt. Im Unterschied zu den zuvor beschriebenen Ausführungsbeispielen dringt das Hülsenelement 40 nicht ganz bis zur Kammerscheidewand des Herzens 25 vor, wenn die Eindringeinrichtung 48 in den Körper 22 eintritt, sondern bleibt von dieser beabstandet. Dies ist in der Figur 8a) zu sehen. Wird nun der Ballon 50 aufgeblasen, so tritt dieser aus dem Hülsenelement 49 aus und schmiegt sich von außen an die Kammerscheidewand des Herzens 25 an. Anstatt wie im vorherigen Ausführungsbeispiel an der Innenwand der Herzkammer 26 anzuliegen, liegt der Ballon 50 im vorliegenden Beispiel nunmehr an der Außenseite des Herzens 25 an der Kammerscheidewand an und steht über diese mit dem Herzen 25 in Kontakt, was in der Figur 8b) zu sehen ist. Stromimpulse können nun mittels des Ballons 50 über die äußere Kammerscheidewand an das Herz 25 abgegeben werden.

Die zuvor beschriebenen Nothilfemodule sind überwiegend zur autarken Verwendung vorgesehen. Eines oder mehrere derartiger Nothilfemodule können aber auch als Teil eines komplexeren Nothilfegerätes vorgesehen werden, das mehrere Funktionen bietet.

Ein derartiges Multifunktionsnothilfegerät 51 ist in der Figur 9 zu sehen. Das Multifunktionsnothilfegerät 51 umfasst ein Gehäuse 52 mit einem Display 53, zwei an dem Gehäuse 51 lösbar befestigte Defibrillatorelektroden 54 sowie ein ebenfalls lösbar am Gehäuse 51 befestigtes erfindungsgemäßes Nothilfemodul 55. Mittels des Displays 53 lässt sich das Multifunktionsnothilfegerät 51 bedienen, das mit den Defibrillatorelektroden 54 wahlweise als Defibrillator oder mit dem Nothilfemodul 55 zur Notfallbehandlung von Asystolien eingesetzt werden kann. Hierbei erfolgt die Steuerung des Nothilfemoduls 55 über eine im Inneren des Gehäuses 52 angeordnete und in der Figur 9 nicht sichtbare Steuereinheit. Um der Steuereinheit nach Lösen des Nothilfemoduls 55 vom Gehäuse 51 eine Kommunikation mit dem Nothilfemodul 55 zu ermöglichen, können gleichermaßen eine Kabelverbindung oder eine drahtlose Kommunikationsverbindung wie zum Beispiel eine Bluetooth- oder eine ähnliche Verbindung zwischen Nothilfemodul 55 und Steuereinheit vorgesehen sein.

Alle der bisher beschriebenen Nothilfemodule 1, 30, 32, 38, 47, 55 können derart abgewandelt werden, dass sie anstatt des intern im Gehäuse 2 angeordneten Medikamentenreservoirs 11 und fallweise auch anstatt der Pumpe 21 oder aber zusätzlich zum Medikamentenreservoir 11 und der Pumpe 21 über Anschlüsse verfügen, mittels denen sie an ein externes Medikamentenreservoir oder eine beliebige externe Medikamentenzufuhr wie zum Beispiel einen oder mehrere Schläuche anschließbar sind. Über diese Anschlüsse können dem Nothilfemodul 1, 30, 32, 38, 47, 55 Medikamente von außen zugeführt werden, die dann auf die bereits beschriebene Weise über die jeweilige Eindringeinrichtung 9, 27, 33, 39, 48 bis zum Herzen 25 gelangen. Dabei kann zur Steuerung der Versorgung mit externen Medikamenten entweder eine externe Steuereinheit vorgesehen sein, oder aber die Steuereinheit 12 des jeweiligen Nothilfemoduls 1, 30, 32, 38, 47, 55 ist für diese Aufgabe eingerichtet. Ferner können neben dem gezeigten Medikamentenreservoir 11 beliebig viele weitere Medikamentenreservoire vorgesehen sein, von denen ein jedes mit einem anderen Medikament gefüllt ist. Medikamente aus diesen Medikamentenreservoiren können jeweils getrennt voneinander der Fluidleitung 20 zugeführt werden, beispielsweise mittels jeweiliger Pumpen, oder aber die Steuereinheit 12 stellt durch Einleiten jeweiliger Medikamente in entsprechenden Mengenverhältnissen in die Fluidleitung 20, wobei zum Beispiel jeweilige Pumpen entsprechend angesteuert werden, ein Medikamentengemisch her, so dass das Herz 25 bedarfsweise entweder mit einem einzelnen der Medikamente oder mit einem jeweiligen Medikamentengemisch versorgt werden kann. Darüber hinaus kann anstelle der Pumpe 21 ein vorzugsweise pneumatisch angetriebener Kolben im Medikamentenreservoir 11 vorgesehen sein, mit dem sich das Medikament aus dem Medikamentenreservoir 11 in die Fluidleitung 20 drücken lässt. Vorzugsweise ist der Hub des Kolbens von der Steuereinheit 12 gesteuert.

Führt die Beaufschlagung des Herzens 25 mit Stromimpulsen zum Erfolg, so beginnt dieses damit, wieder abwechselnd Kontraktions- und Expansionsbewegungen auszuführen. Dabei können Wegunterschiede von bis zum 2 cm von der Herzkammerwand zurückgelegt werden. Mit Einsetzen dieser Bewegungen besteht daher die Gefahr, dass sich die Eindringeinrichtung vom Herzen löst und der Kontakt zwischen der Elektrode der dem Herzen 25 unterbrochen wird. Dies kann mittels einer geeigneten Nachführungseinrichtung vermieden werden, welche dafür sorgt, dass die Eindringeinrichtung den Herzbewegungen folgend nachgeführt wird, um den Kontakt zwischen der Elektrode und dem Herzen auch nach Einsetzen der Herzbewegungen aufrechtzuerhalten.

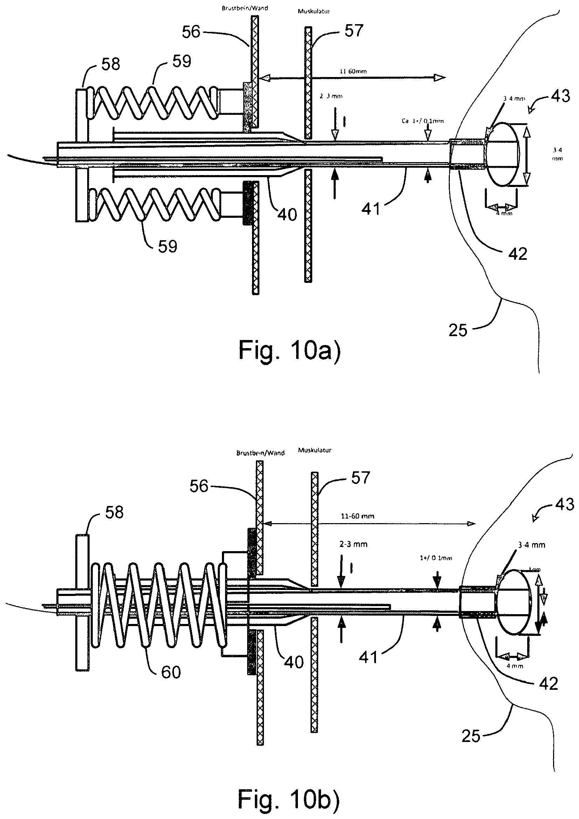

In den Figuren 10a) und 10b) sind Beispiele für derartige Nachführungseinrichtungen zu sehen, die insbesondere für solche Ausführungsformen des Nothilfemoduls vorgesehen sind, bei denen eine interne Fixierung der Eindringeinrichtung im Herzen 25 vorgesehen ist. Dies ist beispielsweise bei den Nothilfemodulen 32 und 38 der Figuren 5c) und 6b) der Fall. Ohne Beschränkung der Allgemeinheit werden die Nachführungseinrichtung und deren Wirkung im Folgenden am Beispiel des Elektrodenelements 41 der Eindringeinrichtung 39 des Nothilfemoduls 38 beschrieben.

Das Elektrodenelement 41 ist in den Figuren 10a) und 10b) das Brustbein 56 und die Muskulatur 57 des Patienten durchstoßend dargestellt, wobei sich die Elektrode 42 wie bereits im Zusammenhang mit der Figur 6b) beschrieben im Inneren der Herzwand befindet und der Ballon 43 aufgeblasen ist. An einem dem Herzen 25 abgewandten Ende des Elektrodenelements 41 ist ein kreisförmiges Plattenelement 58 auf das Elektrodenelement 41 aufgesteckt und an diesem fixiert. Zwischen dem Plattenelement 58 und derjenigen Innenwand des Gehäuses 2 des Nohilfemoduls 38 mit der Öffnung 4 sind Schraubenfedern 59 vorgesehen, die bei Ausfahren des Elektrodenelements 41 aus der Öffnung 4 von dem Plattenelement 58 gestaucht werden. Im Ganzen sind vier derartige Schraubenfedern 59 um das Elektrodenelement 41 herum angeordnet, von denen in der Figur 10a) jedoch lediglich zwei zu sehen sind. Infolge des Stauchens der Schraubenfedern 59 wird von diesen eine Rückstellkraft auf das ausgefahrene Elektrodenelement 41 ausgeübt, die das Elektrodenelement 41 wieder einzufahren trachtet.

Sobald das Herz 25 nun nach Beaufschlagung mit Stromimpulsen wieder zu schlagen beginnt und eine Kontraktionsbewegung ausführt, wird das Elektrodenelement 41 infolge des an der inneren Herzkammerwand anliegenden Ballons 43 weiter aus dem Gehäuse 2 des Nothilfemoduls 38 herausgezogen. Hierdurch werden die Schraubenfedern 59 noch mehr gestaucht und die Rückstellkraft erhöht sich. Während der darauffolgenden Expansion des Herzens 25 sorgt nun diese Rückstellkraft dafür, dass das Elektrodenelement 41 der Herzbewegung folgend wieder in das Gehäuse 2 zurückgezogen wird. Somit stellen die Schraubenfedern 59 und der an der inneren Herzkammerwand anliegende Ballon 43 sicher, dass die Elektrode 42 während der Kontraktions- und Expansionsbewegungen des Herzens 25 den Kontakt zum Herzen 25 nicht verliert.

Im in der Figur 10b) gezeigten Ausführungsbeispiel ist lediglich eine Schraubenfeder 60 vorgesehen, die jedoch anstatt neben dem Elektrodenelement 41 angeordnet zu sein von diesem durchstoßen wird. Im Übrigen ist die Funktionsweise des in der Figur 10b) gezeigten Ausführungsbeispiels analog dem in der Figur 10a) dargestellten Ausführungsbeispiel.

Anstelle der in den Figuren 10a) und 10b) dargestellten Schraubenfedern, die eine Rückstellkraft infolge Stauchung bedingen, kann die Nachführungseinrichtung auch mit Federn ausgeführt sein, die bei Ausfahren der Eindringeinrichtung gedehnt werden und die Rückstellkraft auf die Eindringeinrichtung infolge ihrer Dehnung ausüben. Beispielsweise kann die Eindringeinrichtung mit einer oder mehreren Spiralfedern oder mit einer oder mehreren Zugfedern oder sowohl mit Spiralfeder oder mit Zugfedern ausgestattet sein.

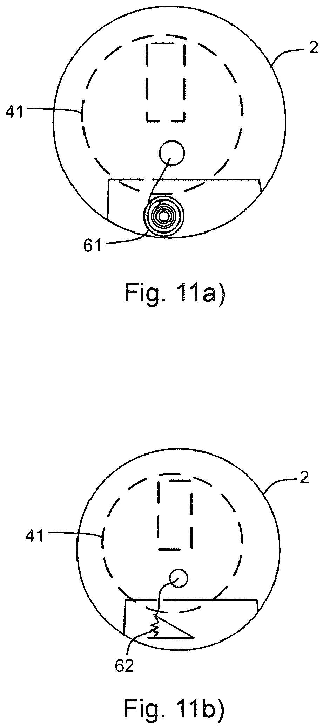

Ein Nothilfemodul mit einer Nachführungseinrichtung, die eine Spiralfeder 61 aufweist, ist in der Figur 11a) in der Draufsicht zu sehen. Die Spiralfeder 61 ist innerhalb des Gehäuses 2 befestigt und mit einem Ende mit der Eindringeinrichtung 41 verbunden. Dringt die Eindringeinrichtung 41 aus dem Gehäuse 2 aus, so wird die am Gehäuse 2 befestigte und mit der Eindringeinrichtung 41 verbundene Spiralfeder 61 gedehnt, wodurch sich eine Rückstellkraft auf die ausgefahren Eindringeinrichtung 41 ergibt, die umso größer ist, je weiter die Eindringeinrichtung 41 ausfährt. Hingegen ist bei dem in der Figur 11b) gezeigten Nothilfemodul die Spiralfeder 61 durch eine Zugfeder 62, beispielsweise eine Schraubenfeder, ersetzt. Im Übrigen ist die Funktionsweise des in der Figur 11b) gezeigten Ausführungsbeispiels analog dem in der Figur 11a) dargestellten Ausführungsbeispiel.

### Bezugszeichenliste

1. Nothilfemodul
2. Gehäuse
3. Anlageelement
4. Öffnung
5. Sensoranordnung
6. Aktivierungstaste
7. rote Leuchtdiode
8. grüne Leuchtdiode
9. Eindringeinrichtung
10. Batterie
11. Medikamentenreservoir
12. Steuereinheit
13. Positionserfassungseinrichtung
14. vorderer Endabschnitt
15. Mechanik
16. Elektrode
17. Durchlass
18. hinteres Ende
19. elektrische Leitung
20. Fluidleitung
21. Pumpe
22. Körper
23. Haut
24. Rippen
25. Herz
26. Herzkammer
27. Eindringeinrichtung
28. Hülsenelement
29. Elektrodenelement
30. Nothilfemodul
31. Elektrode
32. Nothilfemodul
33. Eindringeinrichtung
34. Hülsenelement
35. Elektrodenelement
36. Elektrode
37. Klappelektrode
38. Nothilfemodul
39. Eindringeinrichtung
40. Hülsenelement
41. Elektrodenelement
42. Elektrode
43. Ballon
44. Expansionselement
45. Membran
46. Öffnung
47. Nothilfemodul
48. Eindringeinrichtung
49. Hülsenelement
50. Ballon
51. Multifunktionsnothilfegerät
52. Gehäuse
53. Display
54. Defibrillatorelektrode
55. Nothilfemodul
56. Brustbein
57. Muskulatur
58. Plattenelement
59. Schraubenfeder
60. Schraubenfeder
61. Spiralfeder
62. Zugfeder

## Patentansprüche

1. Vorrichtung (1, 30, 32, 38, 47, 55) zur Beaufschlagung von vorgegebenem Körpergewebe mit Stromimpulsen, die an Körperoberflächen von Organismen anlegbar ist, mit
wenigstens einer Eindringeinrichtung (9, 27, 33, 39, 48), die wenigstens eine Elektrode (16, 31, 36, 42) aufweist, wobei die Eindringeinrichtung (9, 27, 33, 39, 48) dazu vorgesehen ist, nach Anlegen der Vorrichtung (1, 30, 32, 38, 47, 55) an die Körperoberfläche eines Organismus (22) in den Organismus (22) einzudringen und eine Endposition einzunehmen, in der die Elektrode (16, 31, 36, 42) mit dem Körpergewebe (25) des Organismus (22) in Kontakt ist,
wenigstens einer Positionserfassungseinrichtung (13), die dazu vorgesehen ist, eine Position wenigstens eines Teils der an der Körperoberfläche anliegenden Vorrichtung (1, 30, 32, 38, 47, 55) in Bezug auf wenigstens einen Teil des Körpergewebes (25) zu erfassen, und
wenigstens einem Signalausgabemittel (8) zum Ausgeben von Signalen,
**dadurch gekennzeichnet, dass**
die Positionserfassungseinrichtung (13) ferner dazu vorgesehen ist, die erfasste Position daraufhin zu überprüfen, ob sie zum Überführen der Eindringeinrichtung (9, 27, 33, 39, 48) in die Endposition geeignet ist, wobei die Positionserfassungseinrichtung (13) die erfasste Position als geeignet erkennt, wenn eine Strecke von einer vorgesehenen Injektionsstelle für die Eindringeinrichtung (9, 27, 33, 39, 48) bis hin zum Körpergewebe (25) hindernisfrei ist, und
wobei die Vorrichtung (1, 30, 32, 38, 47, 55) eingerichtet ist, in Abhängigkeit von einem Ergebnis der Überprüfung der erfassten Position wenigstens ein Signal auszugeben.

2. Vorrichtung (1, 30, 32, 38, 47, 55) nach Anspruch 1, bei der die Positionserfassungseinrichtung (13) die erfasste Position als geeignet erkennt, wenn sich die erfasste Position zudem innerhalb wenigstens eines vorgegebenen Positionierungsbereichs befindet.

3. Vorrichtung (1, 30, 32, 38, 47, 55) nach Anspruch 1 oder 2, die wenigstens einen Sensor und/oder wenigstens eine Sensoranordnung (5) und/oder wenigstens einen Ultraschallsensor und/oder wenigstens einen Infrarotsensor und/oder wenigstens einen Kraftsensor und/oder wenigstens einen Tiefensensor und/oder wenigstens einen magnetischen Positionssensor und/oder wenigstens einen Lasersensor und/oder wenigstens eine Laserdiode und/oder wenigstens einen Gassensor und/oder wenigstens einen Hautwiderstandssensor und/oder wenigstens einen Sensor zur Impedanzmessung umfasst.

4. Vorrichtung (1, 30, 32, 38, 47, 55) nach einem der vorhergehenden Ansprüche, bei der die Eindringeinrichtung wenigstens ein Schneidmittel umfasst, das zum Aufschneiden des Organismus (22) vorgesehen ist, um der Eindringeinrichtung (9, 27, 33, 39, 48) das Eindringen in den Organismus (22) zu erleichtern.

5. Vorrichtung (30, 32, 38, 55) nach einem der vorhergehenden Ansprüche, bei der die Eindringeinrichtung (27, 33, 39) wenigstens zwei teleskopartig ineinanderschiebbare und auseinanderziehbare Elemente (28, 29, 34, 35, 40, 41) aufweist.

6. Vorrichtung (1, 30, 32, 38, 55) nach einem der vorhergehenden Ansprüche, bei der die Elektrode (16, 31, 36, 42) wenigstens einen Teilbereich einer Außenfläche der Eindringeinrichtung (9, 27, 33, 39, 48) überdeckt oder bildet und/oder bei der die Eindringeinrichtung (33) wenigstens ein aufklappbares Mittel (37) umfasst, wobei die Elektrode wenigstens einen Teilbereich einer Außenfläche des aufklappbaren Mittels (37) bedeckt oder bildet, und/oder bei der die Eindringeinrichtung (39, 48) wenigstens ein expandierbares Mittel (43, 50) umfasst, wobei die Elektrode wenigstens einen Teilbereich einer Außenfläche des expandierbaren Mittels (43, 50) bedeckt öder bildet, und/oder bei der die Eindringeinrichtung (27, 33, 39) wenigstens ein ausfahrbares Teil (29, 35, 41) umfasst, wobei die Elektrode (31, 36, 42) wenigstens einen Teilbereich einer Außenfläche des ausfahrbaren Teils (29, 35, 41) bedeckt oder bildet.

7. Vorrichtung (1, 30, 32, 38, 47, 55) nach einem der vorhergehenden Ansprüche mit wenigstens einer Medikamentenzuführung (20) zur Versorgung des Körpergewebes (25) mit wenigstens einem Medikament und wenigstens einem mit der Medikamentenzuführung (20) gekoppelten Medikamentenreservoir (11) zur Aufnahme von wenigstens einem Medikament und/oder wenigstens einen mit der Medikamentenzuführung (20) gekoppelten Medikamenteneinlass zum Einlassen von wenigstens einem Medikament von außerhalb der Vorrichtung (1, 30, 32, 38, 47, 55).

8. Vorrichtung (1, 30, 32, 38, 47, 55) nach einem der vorhergehenden Ansprüche mit wenigstens einem beweglichen Anlageelement (3) zum Anlegen der Vorrichtung (1, 30, 32, 38, 47, 55) an Körperoberflächen von Organismen (22).

9. Vorrichtung (1, 30, 32, 38, 47, 55) nach einem der vorhergehenden Ansprüche, bei der eine Tiefe, bis zu der die Eindringeinrichtung (9, 27, 33, 39, 48) in den Organismus eindringt, einstellbar ist.

10. Vorrichtung (1, 30, 32, 38, 47, 55) nach einem der vorhergehenden Ansprüche mit wenigstens einer Steuereinheit (12), die zum Steuern der Abgabe von Stromimpulsen an das Körpergewebe (25) und/oder der Überprüfung der erfassten Position und/oder der Ausgabe des Signals und/oder des Eindringens der Eindringeinrichtung (9, 27, 33, 39, 48) in den Organismus (22) und/oder des Überführens der Eindringeinrichtung (9, 27, 33, 39, 48) in die Endposition und/oder des Kontaktierens des Körpergewebes (25) durch die Elektrode (16, 31, 36, 42) und/oder des Aufklappens des aufklappbaren Mittels (37) und/oder des Expandierens des expandierbaren Mittels (43, 50) und/oder der Zuführung von Medikamenten zum Körpergewebe (25) und/oder des Anpassens einer Positionierung des Anlageelementes (3) an eine äußere Form der Körperoberfläche eingerichtet ist.

11. Vorrichtung (1, 30, 32, 38, 47, 55) nach einem der vorhergehenden Ansprüche mit einem Gehäuse (2), wobei die Eindringeinrichtung (9, 27, 33, 39, 48) vom Gehäuse (2) entkoppelbar ist

12. Vorrichtung (1, 30, 32, 38, 47, 55) nach einem der vorhergehenden Ansprüche, die zum Nachführen der Endposition der Eindringeinrichtung (9, 27, 33, 39, 48) eingerichtet ist, wenn das Körpergewebe (25) Bewegungen ausführt.

13. Multifunktionsgerät (51) mit einer Vorrichtung (1, 30, 32, 38, 47, 55) nach einem der vorhergehenden Ansprüche, das ferner wenigstens einen Defibrillator und/oder wenigstens eine Defibrillatorelektrode (54) und/oder wenigstens einen Herzschrittmacher aufweist.

## Claims

1. A device (1, 30, 32, 38, 47, 55) for stimulating a given tissue with electrical impulses that is abuttable against body surfaces of organisms, comprising
at least one puncturing apparatus (9, 27, 33, 39, 48), which has at least one electrode (16, 31, 36, 42), the puncturing apparatus (9, 27, 33, 39, 48) provided to penetrate into an organism (22) after abutting the device (1, 30, 32, 38, 47, 55) against the body surface of the organism (22), and to take a final position where the electrode (16, 31, 36, 42) contacts the tissue (25) of the organism (22);
at least one position determination apparatus (13) provided to determine a position of at least one part of the device (1, 30, 32, 38, 47, 55) abutting against the body surface in relation to at least one part of the tissue (25); and
at least one signal output means (8) for outputting signals;
**characterized in that**
the position determination apparatus (13) is further configured to check the determined position whether it is suitable for transferring the puncturing apparatus (9, 27, 33, 39, 48) into the final position, wherein the position determination apparatus (13) recognizes the determined position as suitable if a route from an intended injection site for the puncturing apparatus (9, 27, 33, 39, 48) to the tissue (25) is free of obstacles, and
wherein the device (1, 30, 32, 38, 47, 55) is configured to output at least one signal in dependence of a result of the check.

2. The device (1, 30, 32, 38, 47, 55) according to claim 1, wherein the position determination apparatus (13) recognizes the determined position as suitable if the determined position is further within at least one preset position range.

3. The device (1, 30, 32, 38, 47, 55) according to any of claims 1 or 2, comprising at least one sensor and/or at least one sensor array (5) and/or at least one ultrasonic sensor and/or at least one infrared sensor and/or at least one force sensor and/or at least one depth sensor and/or at least one magnetic position sensor and/or at least one laser sensor and/or at least one laser-emitting diode and/or at least one gas sensor and/or at least one skin resistance sensor and/or at least one sensor measuring impedance.

4. The device (1, 30, 32, 38, 47, 55) according to any of the previous claims, wherein the puncturing apparatus comprises at least one cutting means for cutting the organism (22) in order to make it easier for the puncturing apparatus (9, 27, 33, 39, 48) to get into the organism (22).

5. The device (30, 32, 38, 55) according to any of the previous claims, wherein the puncturing apparatus (27, 33, 39) has at least two elements (28, 29, 34, 35, 40) that are slideable in a telescope-like way into one another and apart.

6. The device (1, 30, 32, 38, 55) according to any of the previous claims, wherein the electrode (16, 31, 36, 42) covers or forms at least a part of an outer surface of the puncturing apparatus (9, 27, 33, 39, 48); and/or wherein the puncturing apparatus (33) comprises at least one foldable means (37) whereby the electrode covers or forms at least a part of the outer surface of the foldable means (37); and/or wherein the puncturing apparatus (39, 48) comprises at least one expandable means (43, 50) whereby the electrode covers or forms at least a part of the outer surface of the expandable means (43, 50); and/or wherein the puncturing apparatus (27, 33, 39) comprises at least one extendable element (29, 35, 41) whereby the electrode (31, 36, 42) covers or forms at least a part of the outer surface of the extendable element (29, 35, 41).

7. The device (1, 30, 32, 38, 47, 55) according to any of the previous claims, having at least one drug feed (20) for supplying the tissue (25) with at least one drug, and at least one drug reservoir (11) coupled to the drug feed (20) for receiving at least one drug; and/or at least one drug inlet coupled with the drug feed (20) for getting at least one drug from outside the device (1, 30, 32, 38, 47, 55).

8. The device (1, 30, 32, 38, 47, 55) according to any of the previous claims, having at least one movable abutting element (3) for abutting the device (1, 30, 32, 38, 47, 55) against body surfaces of organisms (22).

9. The device (1, 30, 32, 38, 47, 55) according to any of the previous claims, wherein a depth for penetration of the puncturing apparatus (9, 27, 33, 39, 48) into the organism is adjustable.

10. The device (1, 30, 32, 38, 47, 55) according to any of the previous claims, having at least one control unit (12), which is configured for controlling the delivery of electrical impulses to the tissue (25) and/or the checking the determined position and/or the output of signals and/or the penetration of the puncturing apparatus (9, 27, 33, 39, 48) into the organism (22) and/or the transfer of the puncturing apparatus (9, 27, 33, 39, 48) to the final position and/or the contact of the electrode (16, 31, 36, 42) to the tissue (25) and/or the folding-out of the foldable means (37) and/or the expansion of the expandable means (43, 50) and/or the supply of drugs to the tissue (25) and/or the positioning adjustment of the abutting element (3) against an outer form of the body surface.

11. The device (1, 30, 32, 38, 47, 55) according to any of the previous claims, having a housing (2), wherein the puncturing apparatus (9, 27, 33, 39, 48) can be decoupled from the housing (2).

12. The device (1, 30, 32, 38, 47, 55) according to any of the previous claims, configured for tracking the final position of the puncturing apparatus (9, 27, 33, 39, 48) when the tissue (25) performs movements.

13. Multifunctional apparatus (51) having a device (1, 30, 32, 38, 47, 55) according to any of the previous claims, which furthermore has at least one defibrillator and/or at least one defibrillator electrode (54) and/or at least one cardiac pacemaker.

## Revendications

1. Dispositif (1, 30, 32, 38, 47, 55) pour appliquer des impulsions de courant à un tissu corporel donné, qui se laisse épauler à la surface du corps, avec
au moins un dispositif de pénétration (9, 27, 33, 39, 48), qui comporte au moins une électrode (16, 31, 36, 42) et qui est prévu à - après d' avoir épaulé le dispositif (1, 30, 32, 38, 47, 55) à la surface d'un organisme (22) - s'introduire dans l'organisme (22) et à se placer dans une position finale, dans laquelle l'électrode (16, 31, 36, 42) est en contact avec le tissu corporel (25), avec
au moins un dispositif de détection de position (13) qui est prévu à détecter la position au moins d'une part du dispositif (1, 30, 32, 38, 47, 55) en relation à au moins une part du tissu corporel (25) et
avec au moins un moyen (8) pour émettre des signaux,
**caractérisé par le fait que**
le dispositif de détection de position (13) est en outre prévu de vérifier si la position détectée est appropriée à transférer le dispositif de pénétration (9, 27, 33, 39, 48) à la position finale, le dispositif de détection de position (13) acceptant la position détectée comme appropriée si le chemin du lieu d'injection prévu pour le dispositif de pénétration (9, 27, 33, 39, 48) jusqu'au tissu corporel (25) est sans obstacles,
et le dispositif (1, 30, 32, 38, 47, 55) est préparé à émettre au moins un signal en dépendance du résultat de la vérification de la position détectée.

2. Dispositif (1, 30, 32, 38, 47, 55) d'après revendication 1, dont le dispositif de détection de position (13) accepte la position détectée comme appropriée si la position détectée en plus se trouve en l'espace de au moins une région de positionnement donnée.

3. Dispositif (1, 30, 32, 38, 47, 55) d'après revendication 1 ou 2 qui comporte au moins un senseur et/ou au moins arrangement de senseurs (5) et/ou au moins un senseur ultra-sonique et/ou au moins un senseur infrarouge et/ou au moins un senseur de forces et/ou au moins un capteur de profondeur et/ou au moins senseur de position magnétique et/ou au moins un senseur de laser et/ou au moins une diode de laser et/ou au moins un détecteur de gaz et/ou au moins un senseur de résistance cutanée et/ou au moins un senseur pour mesurer des impédances.

4. Dispositif (1, 30, 32, 38, 47, 55) d'après une des revendications précédentes dont le dispositif de pénétration comporte au moins un moyen de couper qui est prévu à éventrer l'organisme (22) pour faciliter au dispositif de pénétration (9, 27, 33, 39, 48) de s'introduire dans l'organisme (22).

5. Dispositif (30, 32, 38, 55) d'après une des revendications précédentes dont le dispositif de pénétration (27, 33, 39) comporte au moins deux éléments (28, 29, 34, 35, 40) qui se laissent entrer l'un dans l'autre et déplier en manière d'un télescope.

6. Dispositif (1, 30, 32, 38, 55) d'après une des revendications précédentes dont l'électrode (16, 31, 36, 42) recouvre ou forme au moins une zone partielle de la surface du dispositif de pénétration (9, 27, 33, 39, 48) et/ou dont le dispositif de pénétration (33) comporte au moins un moyen dépliable (37) dont l'électrode recouvre ou forme au moins une zone partielle de la surface du moyen dépliable (37) et/ou dont le dispositif de pénétration (39, 48) comporte au moins un moyen extensible (43, 50) dont l'électrode recouvre ou forme au moins une zone partielle de la surface du moyen extensible (43, 50) et/ou dont le dispositif de pénétration (27, 33, 39) comporte au moins un moyen escamotable (29, 35, 41) dont l'électrode (31, 36, 42) recouvre ou forme au moins une zone partielle de la surface du moyen escamotable (29, 35, 41),

7. Dispositif (1, 30, 32, 38, 47, 55) d'après une des revendications précédentes avec au moins une amenée de médicaments (20) pour approvisionner le tissu corporel (25) en au moins un médicament et avec au moins un réservoir de médicaments (11) couplé à l'amenée de médicaments (20) pour contenir au moins un médicament et/ou au moins une entrée de médicaments couplée à l'amenée de médicaments (20) pour laisser entrer au moins un médicament de l'extérieur du dispositif (1, 30, 32, 38, 47, 55).

8. Dispositif (1, 30, 32, 38, 47, 55) d'après une des revendications précédentes avec au moins un élément d'épaulement (3) pour épauler le dispositif (1, 30, 32, 38, 47, 55) à des surfaces du corps des organismes (22).

9. Dispositif (1, 30, 32, 38, 47, 55) d'après une des revendications précédentes dont la profondeur à laquelle s'introduit le dispositif de pénétration (9, 27, 33, 39, 48) dans l'organisme se laisse adapter.

10. Dispositif (1, 30, 32, 38, 47, 55) d'après une des revendications précédentes avec au moins une unité de contrôle (12) installée pour contrôler l'application des impulsions de courant au tissu corporel (25) et/ou la vérification de la position détectée et/ou l'émission du signal et/ou la pénétration du dispositif de pénétration (9, 27, 33, 39, 48) dans l'organisme (22) et/ou le transfert du dispositif de pénétration (9, 27, 33, 39, 48) jusqu'à la position finale et/ou le contacter du tissu corporel (25) par l'électrode (16, 31, 36, 42) et/ou le déplier du moyen dépliable (37) et/ou l'extension du moyen extensible (43, 50) et/ou l'application des médicaments au tissu corporel (25) et/ou l'adaption du positionnement de l'élément d'épaulement (3) à une forme extérieure de la surface du corps,

11. Dispositif (1, 30, 32, 38, 47, 55) d'après une des revendications précédentes avec un coffret (2) et dont le dispositif de pénétration (9, 27, 33, 39, 48) se peut découpler du coffret (2).

12. Dispositif (1, 30, 32, 38, 47, 55) d'après une des revendications précédentes qui est préparé à asservir la position finale du dispositif de pénétration (9, 27, 33, 39, 48) quand le tissu corporel (25) se bouge.

13. Appareil multifonctions (51) avec un dispositif (1, 30, 32, 38, 47, 55) d'après une des revendications précédentes qui en outre comporte au moins un défibrillateur et/ou au moins une électrode défibrillateur (54) et/ou au moins un stimulateur cardiaque.
